# EUROPEAN PATENT APPLICATION

(11) **EP 2 604 276 A1**
(43) Date of publication of application: **19.06.2013**
(21) Application number: 11816137.1
(22) Date of filing: 12.08.2011
(51) Int. Cl.: A61K 39/395, A61P 35/00

(54) **USE OF ANTIBODIES AGAINST NK1, NK2 AND/OR NK3 RECEPTORS IN CANCER TREATMENT**

(30) Priority: 12.08.2010 ES 201001062
(71) Applicant: Servicio Andaluz de Salud, 41071 Sevilla (ES)
(72) Inventor: SALINAS MARTIN, Manuel Vicente, 41002 Sevilla (ES)
(74) Representative: Illescas, Manuel
(86) International application number: PCT/ES2011/070595
(87) International publication number: WO 2012/020162

(57) **Abstract**

The object of this invention is the use of an antibody or a fragment thereof specific against NK1, NK2 and/or NK3 receptors of cells, for the manufacture of a medicament for the therapeutic management of human cancer, by direct administration to a mammal, including humans, by inducing cell death or apoptosis of tumour cells, modification of peritumoural microenvironment (consisting of stromal cells, stromal matrix, and intra and peritumoural vascularisation) and/or inflammatory and/or immune response around the tumour. In particular, in the case of melanoma, carcinoma (specially, breast carcinoma, lung, digestive system, prostate, cervix, endometrium, ovary and bladder), leukaemia, lymphomas, neoplasms, such as neuroblastoma and sarcoma, such as osteosarcoma.

SEQUENCE LISTING

<110>  Servicio Andaluz de Salud

<120>  USE OF ANTIBODIES AGAINST NK1, NK2 AND/OR NK3 RECEPTORS IN CANCER
TREATMENT.

<130>  EP-06113

<160>  3

<170>  PatentIn version 3.5

<210>  1
<211>  15
<212>  PRT
<213>  Homo sapiens

<220>
<221>  MISC_FEATURE
<223>  preserved domain corresponding to the carboxyl-terminal region
       of the NK1 receptor

<400>  1

Lys Thr Met Thr Glu Ser Ser Ser Phe Tyr Ser Asn Met Leu Ala
1               5                   10                  15

<210>  2
<211>  86
<212>  PRT
<213>  Homo sapiens

<220>
<221>  MISC_FEATURE
<223>  preserved domain of the NK2 receptor

<400>  2

Arg Phe Arg Ser Gly Phe Arg Leu Ala Phe Arg Cys Cys Pro Trp Val
1               5                   10                  15

Thr Pro Thr Lys Glu Asp Lys Leu Glu Leu Thr Pro Thr Thr Ser Leu
            20                  25                  30

Ser Thr Arg Val Asn Arg Cys His Thr Lys Glu Thr Leu Phe Met Ala
        35                  40                  45

Gly Asp Thr Ala Pro Ser Glu Ala Thr Ser Gly Glu Ala Gly Arg Pro
    50                  55                  60

Gln Asp Gly Ser Gly Leu Trp Phe Gly Tyr Gly Leu Leu Ala Pro Thr
65                  70                  75                  80

Lys Thr His Val Glu Ile

                85

<210>  3
<211>  89
<212>  PRT
<213>  Homo sapiens

<220>
<221>  MISC_FEATURE
<223>  preserved domain of the NK3 receptor

<400>  3

Met Ala Thr Leu Pro Ala Ala Glu Thr Trp Ile Asp Gly Gly Gly Gly
1               5                   10                  15

Val Gly Ala Asp Ala Val Asn Leu Thr Ala Ser Leu Ala Ala Gly Ala
            20                  25                  30

Ala Thr Gly Ala Val Glu Thr Gly Trp Leu Gln Leu Leu Asp Gln Ala
        35                  40                  45

Gly Asn Leu Ser Ser Ser Pro Ser Ala Leu Gly Leu Pro Val Ala Ser
    50                  55                  60

Pro Ala Pro Ser Gln Pro Trp Ala Asn Leu Thr Asn Gln Phe Val Gln
65                  70                  75                  80

Pro Ser Trp Arg Ile Ala Leu Trp Ser
                85

## Description

### TECHNICAL FIELD

This invention is within the field of medicine. More specifically molecular biology applied to medicine, pharmacology and oncology. Specifically, it relates to the use of therapeutic antibodies for the manufacture of medicaments for the treatment of cancer in humans.

### STATE OF THE ART

NK receptors (neuropeptide receptors of Substance P and tachykinins), of NK1, NK2 and NK3 types, are widely distributed in the body cells. Their presence has been shown in the central and peripheral nervous system of mammals, in the digestive system, the circulatory system, hematopoietic and inflammatory and/or immune response cells, as well as in soft tissue, in particular in the vascular endothelium. Multiple biological processes are currently known where NK1, NK2 and NK3 receptors are involved in their regulation.

Substance P is an undecapeptide of natural origin that is produced in mammals, and its sequence was described by Veber et al. (US 4.680.283) and belongs to the family of tachykinins. This family also includes other peptides, such as neurokinin A, neurokinin B, neuropeptide K, neuropeptide gamma and hemokinin I, amongst others. The involvement of Substance P and other tachykinins in the aetiopathogenesis of several diseases has been widely reported in the scientific literature. In this regard, the action of tachykinins has been related to the aetiopathogenesis of human nervous system diseases, such as Alzheimer's disease, multiple sclerosis, Parkinson's disease, anxiety, and depression (Barker R. et al., 1996; Kramer MS, et al. 1998).

The involvement of tachykinins has been also been evidenced in the ethiopathogenesis of several diseases with inflammatory component, such as rheumatoid arthritis, asthma, allergic rhinitis, inflammatory bowel diseases, such as ulcerative colitis and Crohn's disease (Maggi CA, et al. 1993).

In this sense, non-peptide antagonists of NK1 receptors have been developed as medicaments for the treatment of several central nervous system disorders, such as depression, psychosis and anxiety (WO 95/16679, WO 95/18124, WO 95/23798, and WO 01/77100). It has been described that the use of selective NK1 receptor antagonists is useful for the treatment of nausea and vomiting induced by anticancer chemotherapy agents, as well as for the treatment of some forms of urinary incontinence (Quartara L. et al., 1998; Doi T. et al., 1999).

In a study published in 2003 (Giardina G, et al., 2003), a review was made of the most recent patents on NK1, NK2 and NK3 receptor antagonists. The molecules from the most important world manufacturers are described, indicating their possible applications, including mainly: antidepressive, anti-inflammatory, anxiolytic, antiemetic, treatment of ulcerative colitis, and other.

The article published by Antal Orosz et al. (Orosz A, et al., 1995) describes the use of several Substance P (PS) antagonists to inhibit proliferation of lung cell carcinoma (for instance, in cells designated as NCI-H69). On the other hand, the article published by Bunn PA Jr (Bunn PA Jr. et al., 1994) describes Substance P antagonists that can inhibit growth *in vitro* of several commercial cells lines of lung cancer (for instance, the cells designated as NCI-H510, NCI-H345 and SHP-77).

In the article published by Palma C et al. (Palma C. et al., 2000), it is described that the astrocytes of the central nervous system express functional receptors for several neurotransmitters, including NK1 receptors. In brain tumours, malignant glial cells derived from astrocytes trigger under the action of tachykinins and by mediation of NK1 receptors the secretion of mediators increasing their proliferation rate. Consequently, selective NK1 antagonists can be very useful as therapeutic agents for the treatment of malignant gliomas.

Patent EP 773026 (Pfizer) makes reference to the use of non-peptide NK1 receptor antagonists for the treatment of cancer in mammals. In particular in the treatment of small lung carcinoma, APUDOMAS (Amine Precursor Uptake and Decarboxylation, tumours located mainly in the digestive tube mucosa), neuroendocrine tumour, and small extra-pulmonary carcinoma.

On the other hand, patent WO 2001001922 describes the use of NK1 receptor antagonists for the treatment of adenocarcinoma and very specifically prostate carcinoma.

Several studies with specific NK1 and NK2 antagonists (such as, CP-96341-1-Pfizer-, MEN 11467, SR 48968 -Sanofi- and MEN 11420 -nepadutant-) have shown their efficacy in blocking cell proliferation (Singh D et al., 2000; and Bigioni M. et al., 2005).

It has been described that non-peptide NK receptor antagonists induce apoptosis (cell death) in tumour cells from various tumours, such as stomach carcinoma, colon carcinoma (Rosso M, et al., 2008) or melanoma (Muñoz M, et al. 2010). Furthermore, it has been described that these receptors are over-expressed in cancer cells.

Patent ES 2 246 687 claims the "use of non-peptide NK1 receptor antagonists and Substance P for the manufacture of a pharmaceutical composition for the induction of apoptosis in tumour cells from mammals" and, therefore, "for the treatment of cancer".

Antibodies are peptide compounds with ability for a highly selective binding to several organic molecules. Their binding to some cell receptors induces a modification in the activity thereof, which can lead to a change in the regular physiological activities of cell metabolism.

The use of monoclonal antibodies to the extracellular domain of several cell receptors, such as the treatment of cancer, is widespread in clinical practice. These therapeutic monoclonal antibodies approved for use in oncology include mainly trastuzumab, which is an anti-ErbB2/HER2 for breast cancer, cetuximab, which is an anti-ErbB1/EGFR for colon cancer, and bevacizumab, which is an anti-VEGFR (vascular endothelium growth factor receptor) for several types of cancer (Adams GP. et al., 2005).

Patent application WO/2009/125039 claims the "Use of an antibody or fragment thereof, specific against Substance P, for the manufacture of a medicament for the therapeutic management of cancers expressing the NK1 receptor and/or a member of the ErbB receptors family, by direct administration to a mammal, including humans".

In addition, in the genesis and development of cancer not only the molecular mechanisms of tumour cells are involved, as stromal cells, inflammation and interactions between tumour cells and those of the stroma and inflammation are also very important (McAllister SS, et al. 2010; Ikushima H, Miyazono K, 2010).

In addition, some substances, for instance NF-Kb (nuclear factor kappa B), TGF-beta (transforming growth factor beta) and SPARC (secreted protein, acidic, cysteine-rich) or TGF-alpha (transforming growth factor alpha), have been shown to be present in the tumour microenvironment and are very important in the genesis and progression of tumours (Coussens L and Werb Z. 2002). TGF-beta can inhibit the transformation from precursor forms of CD8+ lymphocyte to effector cell forms (Berzofsky JA, et al. 2004). SPARC plays a major role in tumour neoangiogenesis (Carmeliet P and RK Jain. 2000). Neoangiogenesis, immunity, and inflammation are key factors for tumour progression (Hanahan D and Weinberg RA. Cell. 2000).

The importance of some molecules produced by fibroblasts in cancer progression is well known and the use of them as therapeutic targets has been even proposed for the treatment of cancer by the use of monoclonal antibodies specific against them (Welt S, et al. 1994).

Specifically, in relation to peritumoural microenvironment, metalloprotases (MMPs) are enzymes containing a metal (zinc) and degrading the extracellular matrix proteins (collagen IV, laminins, elastins, fibronectins, proteglycans, etc). They are expressed physiologically in some situations, such as wound healing, transition from cartilage to bone, and placental development. They are expressed pathologically in the process of invasion and development of metastasis in tumours (Cox G. et al. 2000). The MMPs most commonly involved in tumour development are: MMP-2 (Gelatinase A), MMP-3 (Stromelysin 1), MMP-7 (Matrilysin), MMP-9 (Gelatinase B), MMP-11 (Stromelysin 3), MMP-13 (Collagenase 3), and MMP-14. There are several drugs known as metalloprotease inhibitors that are currently tested for the treatment of cancer: Batimastat (peptidomimetic with MMP-1,2,3,7 and 9 as targets), Marimastat (peptidomimetic with MMP-1,2,3,7,9 and 12 as targets), Prinomastat (non-peptidomimetic, with MMP-2,3,9,13 and 14 as targets), Bay-129566 (non-peptidomimetic, with MMP-2,3 and 9 as targets), Metastat (tetracycline with MMP-2 and 9 as targets), BMS 275291 (non-peptidomimetic with MMP-2 and 9 as targets) and Neovastat (obtained from shark cartilage, with MMP-1,2,7,9,12 and 13 as targets). Specifically, Marimastat has completed phase I clinical trials in cancer of the breast and non-small cell of the lung, Prinomastat has completed phase I clinical trials in prostate cancer, Bay-129566 has completed phase I clinical trials in several solid tumours, and BMS 275291 has completed phase I clinical trials in non-small cell lung cancer. In addition, it has been reported that the use of Marimastat has been shown to be effective for the treatment of advanced pancreatic cancer(Rosemurgy et al., Procc ASCO 1999), for the treatment of advanced gastric cancer (Fieldng et al., Procc ASCO 2000), for glioblastoma (Puphanich et al., Procc ASCO 2001), and for breast cancer after first-line chemotherapy treatment (Sparano et al., Procc ASCO 2002).

Therefore, in conclusion, the following facts are currently known in the state of the art:
1. NK1, NK2 and NK3 receptors are widely diffused in the human body.
2. Tachykinins and specifically Substance P act on the NK1 receptor.
3. Non-peptide NK1 receptors antagonists can be used for the manufacture of a medicament for the treatment of several central nervous system disorders, such as depression, psychosis and anxiety, which has been object of claim in several patent applications (WO 95/16679, WO 95/18124, WO 95/23798, and WO 01/77100).
4. These receptors are over-expressed in cancerous cells, compared to normal non-cancerous cells of the human body.
5. The use of non-peptide NK1 receptor antagonists has shown effects on tumour cells, resulting in apoptosis (cell death) thereof. In this regard, the "use of non-peptide NK1 receptor antagonists and Substance P for the manufacture of a pharmaceutical composition for production of apoptosis in mammal tumour cells" and, therefore, "for the treatment of cancer" has been claimed in patent ES 2 246 687.
6. The "direct administration of a substance P antibody to different tumour cell lines reduces the proliferation of the tumour cell and induces death thereof", as set out in the patent application WO/2009/125039, that claims the "use of an antibody or fragment thereof, specific against substance P for the manufacture of a medicament for the therapeutic management of cancers expressing the NK1 receptor and/or a member of the family of ErbB receptors, by direct administration to a mammal, including humans".
7. That, as stated, patent ES 2 246 687 claims the use of non-peptide NK1 receptor antagonists and substance P (and lists a given number of them, specifically). Therefore, it does not include antibodies or fragments derived thereof, which are obviously and clearly of peptide nature. This has allowed, for instance, processing the patent application WO/2009/125039, that claims the use of an antibody or fragment thereof, specific against substance P for the therapeutic management of cancer. This is so because obviously, as discussed in the above section, antibodies are of peptide nature, which has prevented the existence of a conflict between the two patents (ES 2 246 687 and WO/2009/125039).
8. The presence of NK1, NK2 and/or NK3 receptors has been demonstrated in the blood cells involved in the inflammatory and/or immune response, in stromal matrix cells and in the cells of vascularisation around the tumour cells. It is known that stromal cells, the blood cells involved in inflammatory and/or immune response and the cells of vascularisation influence tumour progression.

However, the known prior art, including using non-peptide NK1 receptors antagonists and an antibody or fragment thereof, specific against substance P, for the treatment of cancer, the object of this invention as well as the technical advantage contributed, is the use of an antibody or fragment thereof aimed against NK1, NK2 and/or NK3 cell receptors, for the manufacture of a medicament or a pharmaceutical composition for the therapeutic management of cancer by direct administration to a mammal, including humans, as these antibodies can modify the activity of these receptors, inducing tumour cell apoptosis, changes in peritumoural cells and modifications in the inflammatory and/or immune response, with beneficial therapeutic effects.

### DESCRIPTION OF THE INVENTION

### Brief description of the invention

The object of this invention is the use of an antibody or a fragment thereof specific against NK1, NK2 and/or NK3 cell receptors, for the manufacture of a medicament for the therapeutic management of human cancer, by inducing cell death or apoptosis of tumour cells, modification of peritumoural microenvironment (consisting of stromal cells, stromal matrix, and intra and peritumoural vascularisation) and/or inflammatory and/or immune response around the tumour. In particular in the case of melanoma, carcinomas (particularly, lung carcinoma, breast carcinoma, digestive system carcinoma, prostate carcinoma, thyroid carcinoma, cervix carcinoma, endometrium carcinoma, ovary carcinoma, bladder carcinomaand choriocarcinoma), adenocarcinoma (particularly, colon adenocarcinoma, breast adenocarcinoma, ovary adenocarcinoma, pancreas adenocarcinoma, lung adenocarcinoma, digestive system adenocarcinoma), leukaemia (particularly, human lymphoblastic leukaemia), lymphomas (particularly human B-cells lymphomas or T-cells lymphomas, Hodgkin lymphoma and Burkitt lymphoma), neoplasms of glial type, neoplasms of neuroectodermic origin, human Edwing sarcoma, other sarcomas (such as rhabdomyosarcoma and osteosarcoma) and other neoplasms of embryonic origin, such as neuroblastoma, medulloblastoma and nephroblastoma.

NK1, NK2 and/or NK3 receptor antibodies are highly specific antagonists against these receptors, modifying or blocking the activity thereof and inducing death and apoptosis of tumour cells, as well as changes in stromal cells and in the stromal matrix, around these tumour cells, changes in the cells involved in inflammation and immunity, in addition to changes in the vascularisation of tumour and peritumoural cells (which form the tumoural microenvironment).

In brief, these changes in the tumoural microenvironment can be summarised in the modification of the fibroblast immunophenotype (in particular with regard to the production of metalloproteases, NF-Kb, TGF-beta and SPARC - key elements in tumour progression-), the modification of the immunophenotype of inflammatory cells (for instance, TGF-beta and NF-kB - that also are very important in tumour progression-) and inhibition of vascular endothelial cell proliferation (neoangiogenesis is a key determinant factor of tumour progression).

NK1, NK2 and NK3 receptor antibodies induce apoptosis in several commercial tumoural cell lines. In addition they induce changes in the cells forming the tumoural microenvironment that are beneficial for the treatment of cancer: in fibroblasts, in inflammation cells (mononuclear and polymorphonuclear) and in the vascular endothelium. Therefore, NK1, NK2 and NK3 receptor antibodies not only induce death by apoptosis in tumour cells, but also revert changes in cells of the microenvironment (stroma -fibroblasts-, vessels -vascular endothelium- and inflammatory cells) that enhance growth and perpetuation of tumours by interaction between stromal and cancer cells.

Tumour cell death and changes in the tumoural microenvironment lead to a reduction of tumour size or total elimination of tumours. Therefore, this invention relates to the use of an antibody or fragment thereof, against NK1, NK2 and/or NK3 receptors by direct administration to humans, for the treatment of cancer.

The invention also comprises a method for the therapeutic treatment of cancer in humans, that comprises the administration of an effective amount of an antibody or fragment thereof against NK1, NK2 and/or NK3 receptors, which modifies their activity in cells.

In this invention, the term "direct administration" means that the treatment is not given by immunisation with an immunogenic form of NK1, NK2 and/or NK3 receptors for the production of antibodies by the patient. In this invention, the antibody is produced out of the body and is administered to the patient.

The treatment proposed in this invention is useful for patients with cancer, who are asymptomatic, symptomatic, on neoadjuvant treatment (pre-surgery treatment), on adjuvant treatment (supplemental treatment after surgery, when there is no detectable gross tumour) and on treatment of the disease in metastatic stage.

"Cancer" is defined as a malignant tumour of unlimited growth potential expanding locally by invasion and systemically by metastasis. According to this invention, the NK1, NK2 and/or NK3 receptor antibody or fragment thereof is administered to subjects with cancer.

The tumour cells on which NK1, NK2 and/or NK3 receptor antibodies act are selected among primary cells of human invasive malignant melanoma, metastatic cells of human melanoma, human melanoma cells located in the lymph nodes, human glioma cells, human breast carcinoma cells, human small-cell lung carcinoma cells, human large-cell lung carcinoma cells, human pancreatic carcinoma cells, human prostate carcinoma cells, human colon carcinoma cells, human stomach carcinoma cells, human ovary carcinoma cells, human endometrial carcinoma cells, human cervix carcinoma cells, human thyroid carcinoma cells, human bladder carcinoma cells, human choriocarcinoma cells, human acute B cell lymphoblastic leukaemia cells, human acute T cell lymphoblastic leukaemia cells, human Burkitt lymphoma cells, human Hodgkin lymphoma cells, human neuroblastoma cells, human retinoblastoma cells, human Ewing sarcoma cells, human rhabdomyosarcoma cells, and human osteosarcoma cells, amongst others (see Table 1).

As used herein, "NK1, NK2 and/or NK3 receptor antibody" means any polyclonal or monoclonal antibody against NK1, NK2 and/or NK3 receptors. The invention also considers the use of fragments of these antibodies. An antibody fragment means a part of a NK1, NK2 and/or NK3 receptor antibody that has a sufficient size and adequate structure to bind to an epitope present in NK1, NK2 and/or NK3 receptors as well as modifying its normal function, including avoiding that substance P or other agonists of these receptors bind to the aforementioned receptors.

As used herein, the term "specific" means that the antibody has no random associations with other antigens. Multiple well-known assay techniques based on immune reactions between antigens and antibodies can be used with NK1, NK2 and/or NK3 receptors, as antigen to establish whether a given antibody or a fragment thereof has the ability to bind to an epitope present in NK1, NK2 and/or NK3 receptors, and for potentially modifying their activity. These techniques can be, for example, "enzyme-linked immunosorbent assays" (ELISA), immunofluorescence assays (IFA), radioimmunoassays, immunoelectrophoresis, and inmunoblotting. In addition, to establish whether a given antibody or an antibody fragment has the ability to treat cancer, an apoptosis induction assay may be used, that includes staining with DAPI (4'-6-diamidine-2-phenylindol) or with annexin.

Another object of this invention is the use of an antibody or fragment thereof to modify the function of NK1, NK2 and/or NK3 receptors in the production of a pharmaceutical composition or a medicament for the treatment of cancer. It must be understood that the pharmaceutical composition or medicament against NK1, NK2 and/or NK3 receptors is in a pharmaceutically acceptable form to be administered to an individual directly, preferably by intravenous, oral, parenteral or any other route. Intravenous administration refers directly to the application of the antibody or fragment thereof or a pharmaceutical composition comprising it, directly into the bloodstream of the patient. Oral administration can involve swallowing, so that the antibody or fragment thereof, as well as a pharmaceutical composition comprising it contacts the gastrointestinal tract, or oral or sublingual administration can be used, where the compound enters the bloodstream directly from the mouth. Parenteral administration refers to administrations routes other than enteric, transdermal or by inhalation and typically is by injection or infusion. Parenteral administration includes intravenous, intramuscular and/or subcutaneous injection or infusion.

In addition, the antibody or fragment thereof, against NK1, NK2 and/or NK3 receptors of the invention, can be administered alone or in a composition with pharmaceutically acceptable carriers and/or excipients. A person skilled in the art will adapt the composition depending on the particular method of administration. In case a purified antibody is administered, oral administration is the preferred method and is achieved preferably through solid dosage forms, including capsules, tablets, pills, powder and granules, amongst others, or for liquid dosage forms. The preparations of the NK1, NK2 and/or NK3 receptor antibody for parenteral administration preferably include aqueous or non-aqueous sterile solutions, suspensions or emulsions, amongst others.

The term "pharmaceutically acceptable carriers or vehicles" relates to a vehicle that must be approved by a regulatory agency of the federal government or a state government or is listed in the US Pharmacopeia or the European Pharmacopoeia, or another pharmacopoeia generally recognised for use in animals, more specifically in humans. Furthermore, adequate pharmaceutically acceptable excipients will change depending on the particular dosage form selected. In addition, adequate pharmaceutically acceptable excipients can be selected for a particular function they can have in the composition. For instance, some pharmaceutically acceptable excipients can be selected for their ability for enhancing the production of uniform dosage forms. Some pharmaceutically acceptable excipients can be selected for their ability for enhancing the production of uniform dosage forms. Some pharmaceutically acceptable excipients can be selected for their ability to enhance transport of the compound(s) of the invention once administered to the patient from one organ or part of the body to another organ or part of the body. Some pharmaceutically acceptable excipients can be selected for their ability for enhancing acceptance by the patient. The adequate pharmaceutically acceptable excipients include the following types of excipients, without excluding others known in the state of the art: diluents, loads, binding agents, disintegrating agents, lubricants, gliding agents, granulation agents, coating agents, moisturising agents, dissolvents, co-dissolvents, suspension agents, emulsifiers, sweeteners, flavouring agents, taste-masking agents, colouring matters, agents against formation of cakes, wetters, chelating agents, plasticisers, viscosity enhancers, antioxidants, preservatives, stabilisers, surfactants, and buffering agents. The expert in the art shall notice that some pharmaceutically acceptable excipients can fulfil more than one function and can perform alternative functions depending on the amount of excipient present in the formulation and the other ingredients present in the formulation. Experts have the knowledge and the skills in the art allowing them to select adequate pharmaceutically acceptable excipients in the appropriate amounts for use in the invention. In addition, several resources are available for the expert in the art that describe pharmaceutically acceptable excipients, and can be useful for the selection of adequate pharmaceutically acceptable excipients. Examples include the Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The dosage of the active ingredient may be selected depending on the intended therapeutic effect, the administration route, and the duration of treatment. The dose of administration and the frequency shall depend on the size, age and health conditions of the subject, considering the possibility of side effects. The administration shall also depend on the concomitant treatment with other drugs and the tolerance of each subject to the drug administered. Experts in the art may establish the appropriate dose using standard procedures. It is understood that the dose should be the effective amount of antibody or fragment thereof, as the treatment should have at least the same or better effect than the current therapeutic doses in these patients.

The composition may comprise the antibody or fragment thereof as single agent against cancer or combinations thereof with other therapeutic agents, depending on the condition.

Unless otherwise defined, all technical and scientific terms used herein have the same meaning as those usually understood by an expert in the field of the invention. Methods and materials similar or equivalent to those described herein can be used in the practice of this invention. In the description and in the claims, the word "comprises" and its variants does not intend to exclude other technical characteristics, additives, components or steps. Those skilled in the art will conclude other objects, advantages and characteristics of the invention, in part from the description and in part from the practice of the invention.

### Description of the figures

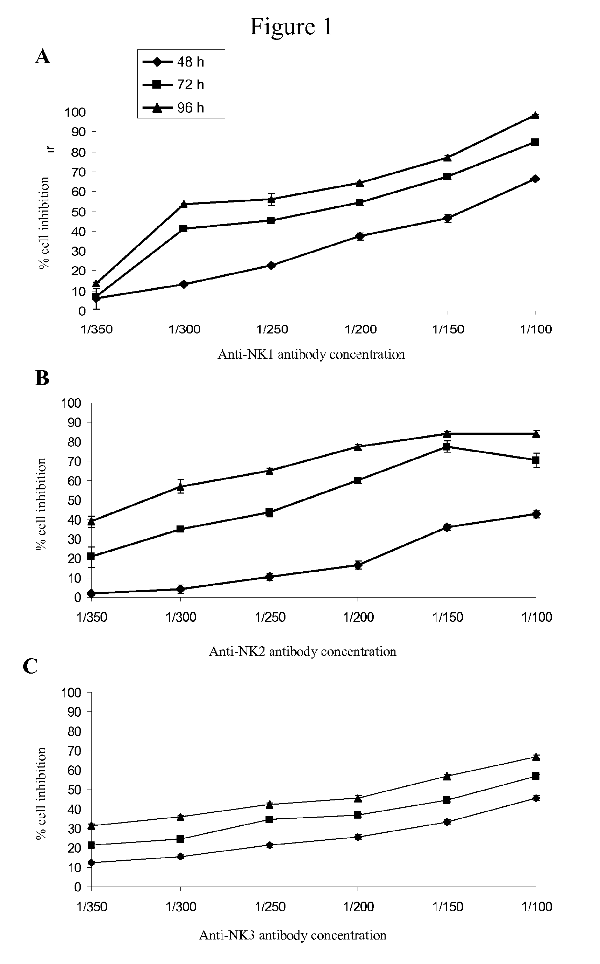

Figure 1. Inhibition of proliferation (expressed as percentage with respect to control cultures) in the commercial cell line of human adenocarcinoma of the colon (SW-403) obtained from the collection of cell lines of the company DSMZ (Deutsche Sammlung von Mikrorganismen und Zellkultures) and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

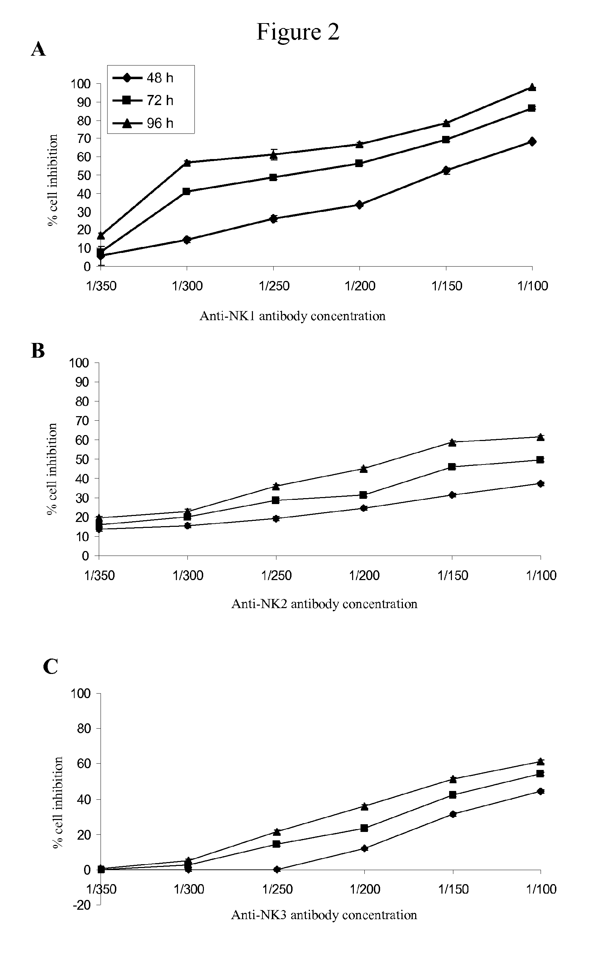

Figure 2. Inhibition of proliferation (expressed as percentage with respect to control cultures) in the commercial cell line of human adenocarcinoma of the breast (MCF-7) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

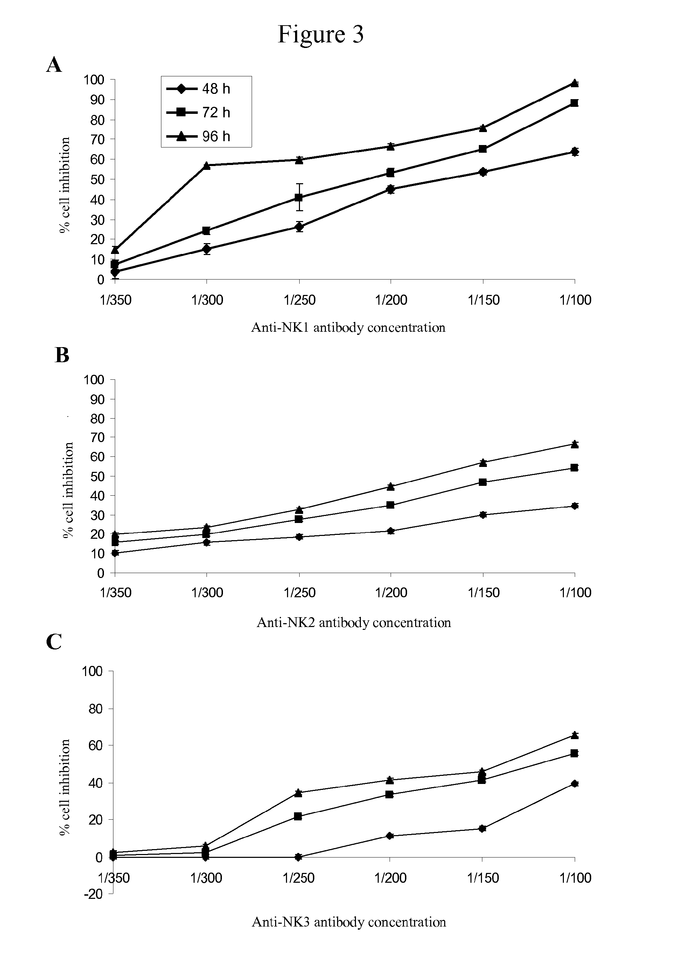

Figure 3. Inhibition of proliferation (expressed as percentage with respect to control cultures) in the commercial cell line of human adenocarcinoma of the ovary (EFO-27) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

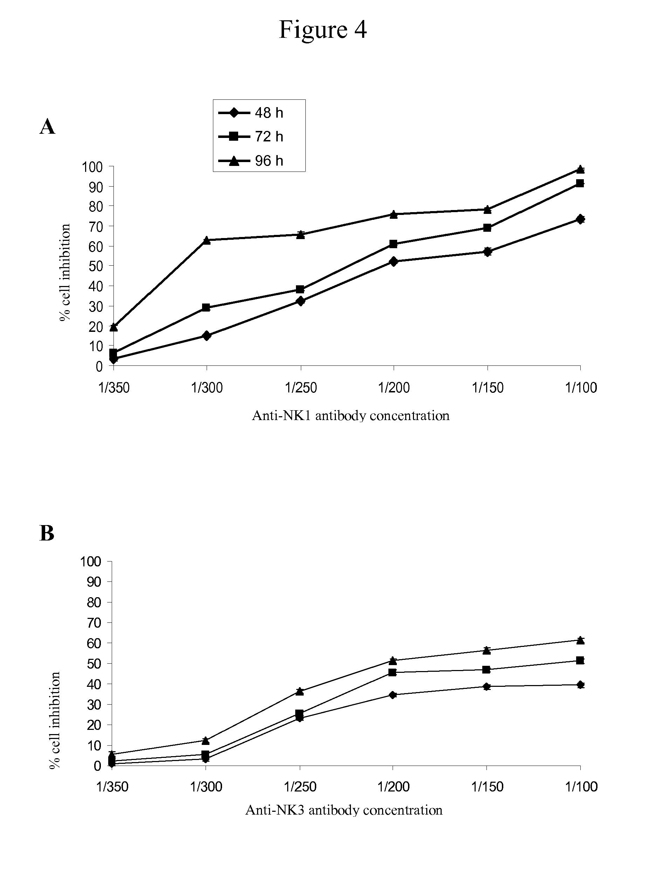

Figure 4. Inhibition of proliferation (expressed as percentage with respect to control cultures) in the commercial cell line of human adenocarcinoma of the pancreas (CAPAN-1) obtained from the collection of cell lines of the company DSMZ and cultured under the conditions set out by this company, by treatment with anti-NK1 (A) or anti-NK3 (B) antibodies to antibody titres shown in the graph and for 48, 72 and 96 hours. As control of proliferation, the cells were cultured in the absence of the aforementioned antibodies.

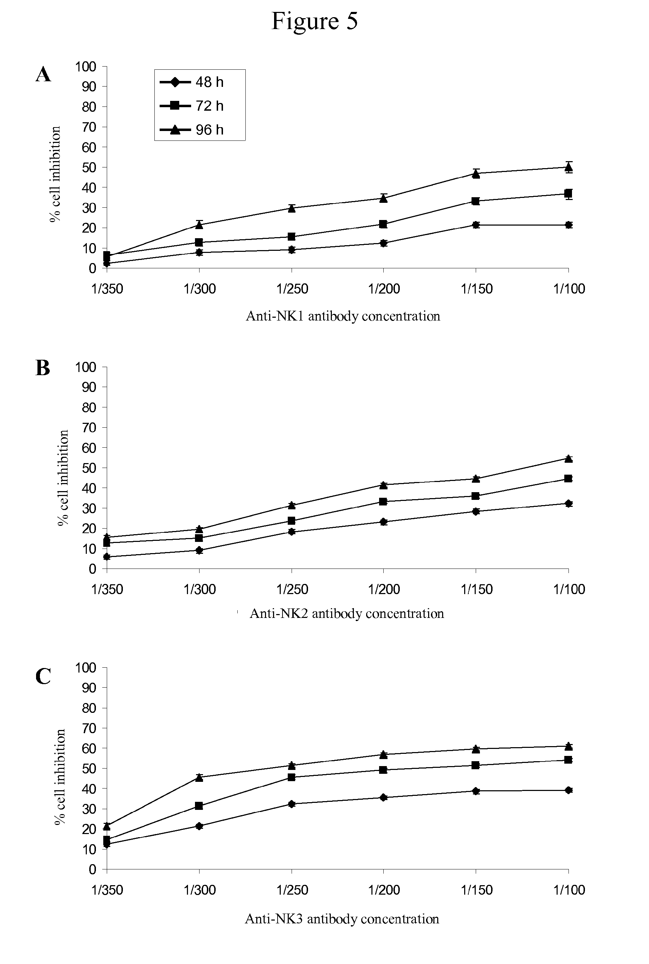

Figure 5. Inhibition of proliferation (expressed as percentage with respect to control cultures) in the commercial cell line of human adenocarcinoma of the pancreas (PA-TU-8902) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

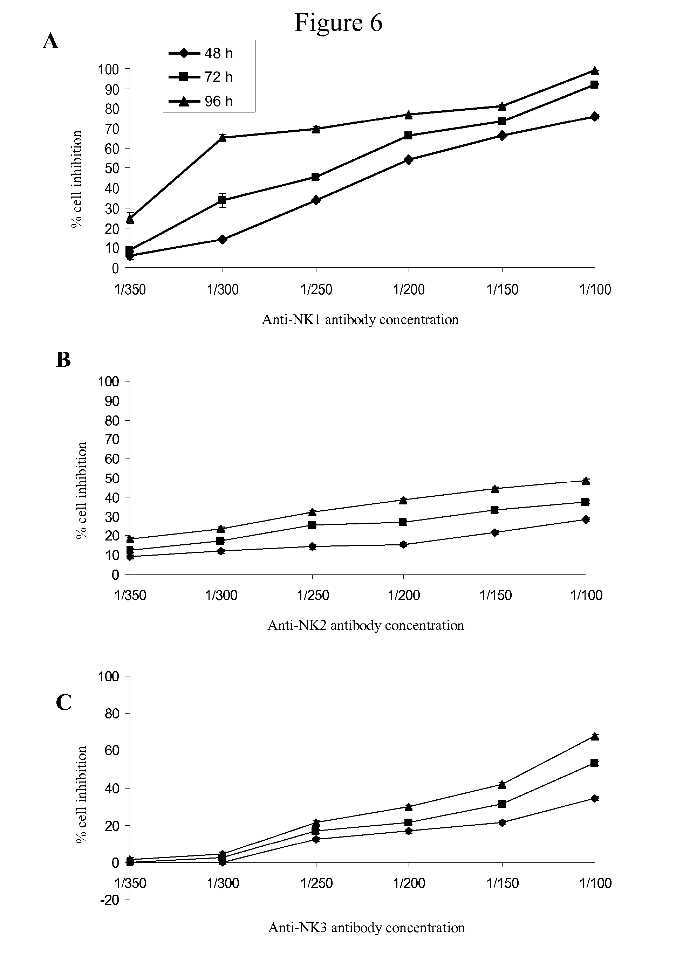

Figure 6. Inhibition of proliferation (expressed as percentage with respect to control cultures) in the commercial cell line of human adenocarcinoma of the lung (DV-90) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

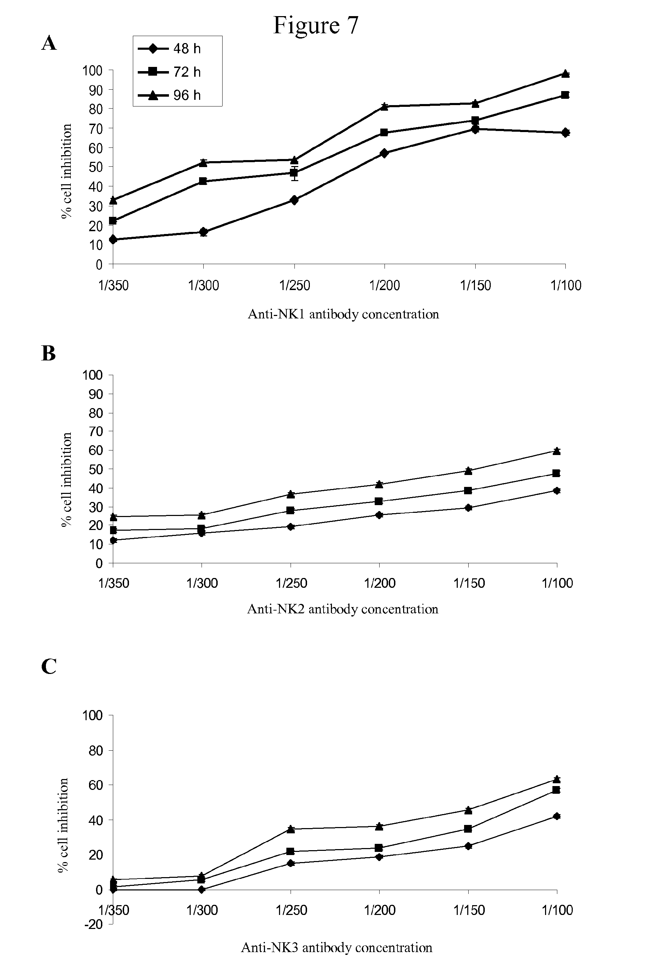

Figure 7. Inhibition of proliferation (expressed as percentage with respect to control cultures) in the commercial cell line of human gastric adenocarcinoma (23132/87) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

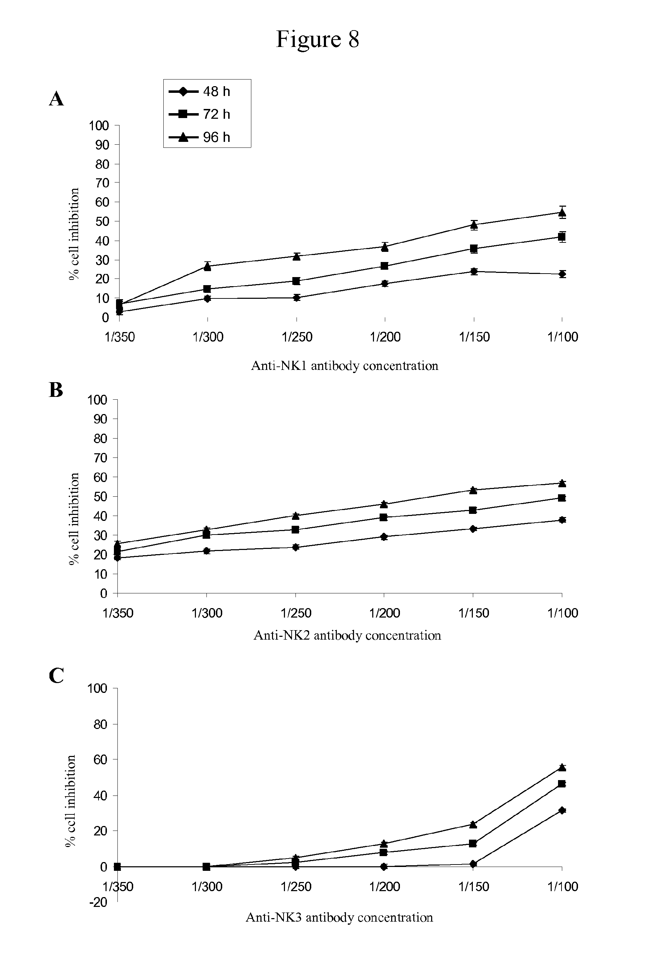

Figure 8. Inhibition of proliferation (expressed as percentage with respect to control cultures) in the commercial cell line of human cervix carcinoma (KB) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

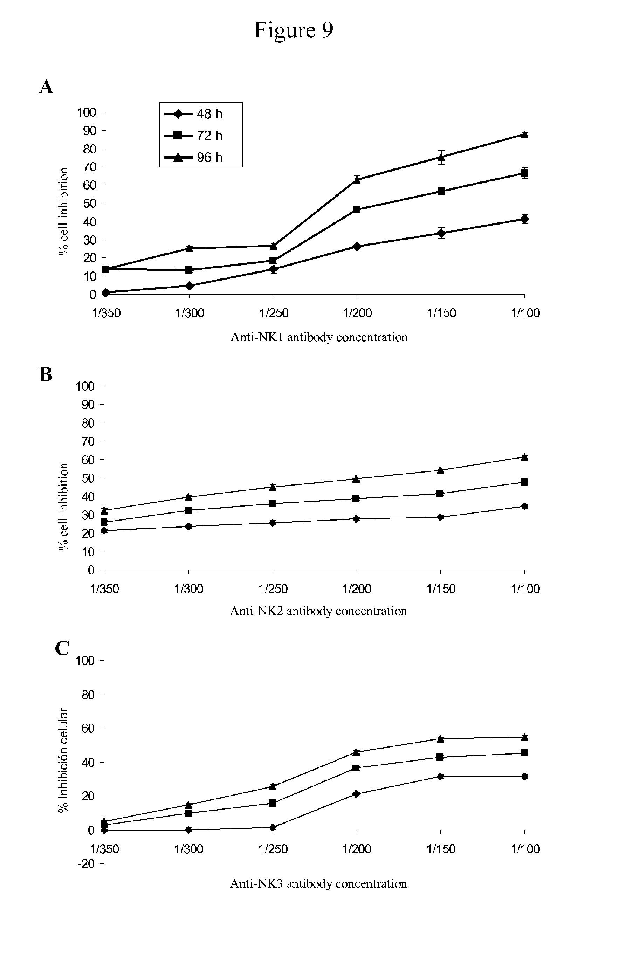

Figure 9. Inhibition of proliferation (expressed as percentage with respect to control cultures) in the commercial cell line of human cervix carcinoma (BT-B) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

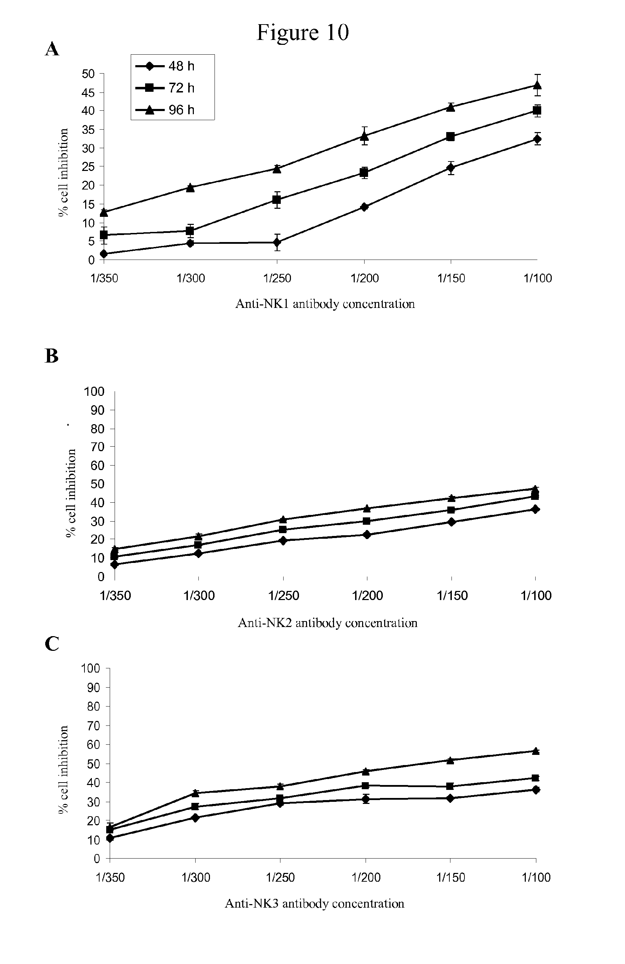

Figure 10. Inhibition of proliferation (expressed as percentage with respect to control cultures) in the commercial cell line of human endometrial carcinoma (AN3-CA) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

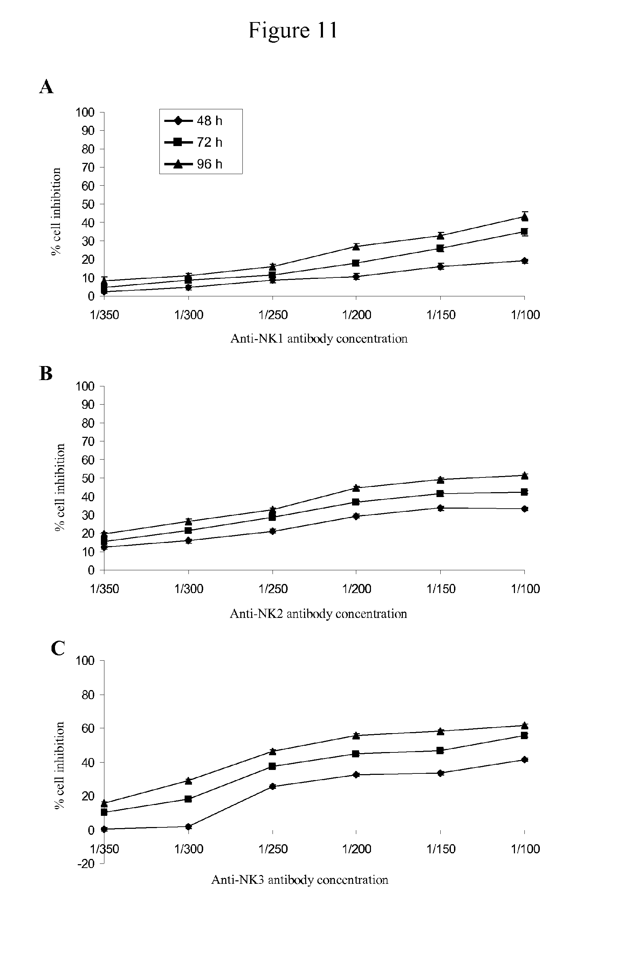

Figure 11. Inhibition of proliferation (expressed as percentage with respect to control cultures) in the commercial cell line of human breast carcinoma (MT-3) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

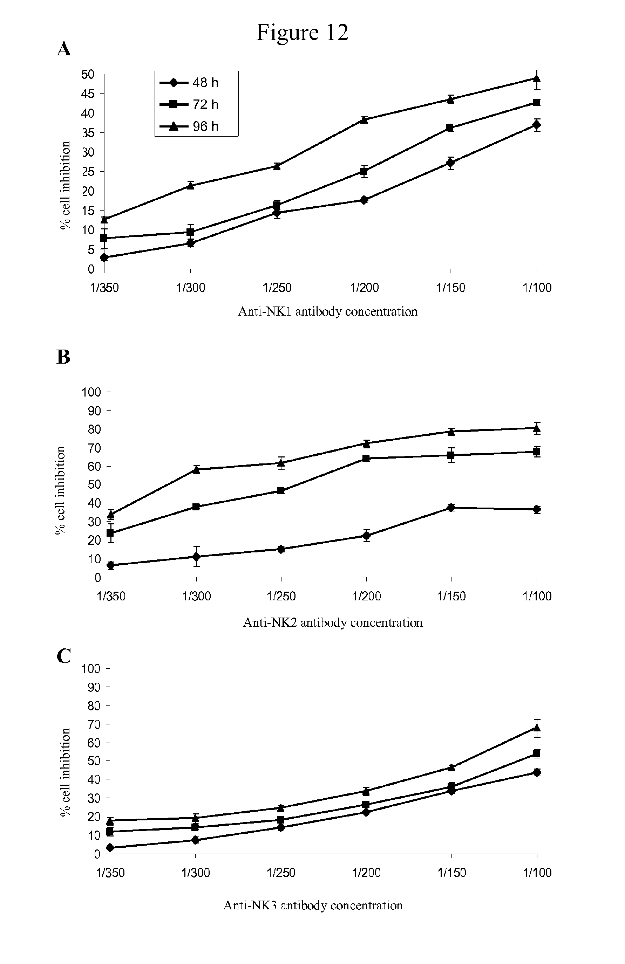

Figure 12. Inhibition of proliferation (expressed as percentage with respect to control cultures) in the commercial cell line of human breast carcinoma (MDA-MB-468) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

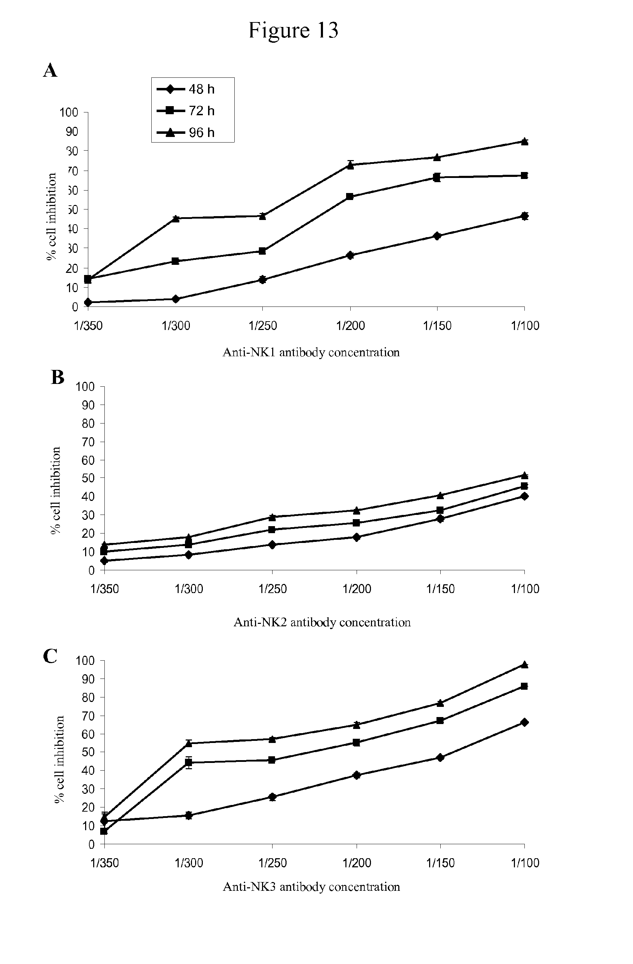

Figure 13. Inhibition of proliferation (expressed as percentage with respect to control cultures) in the commercial cell line of human carcinoma of the ovary (COLO-704) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

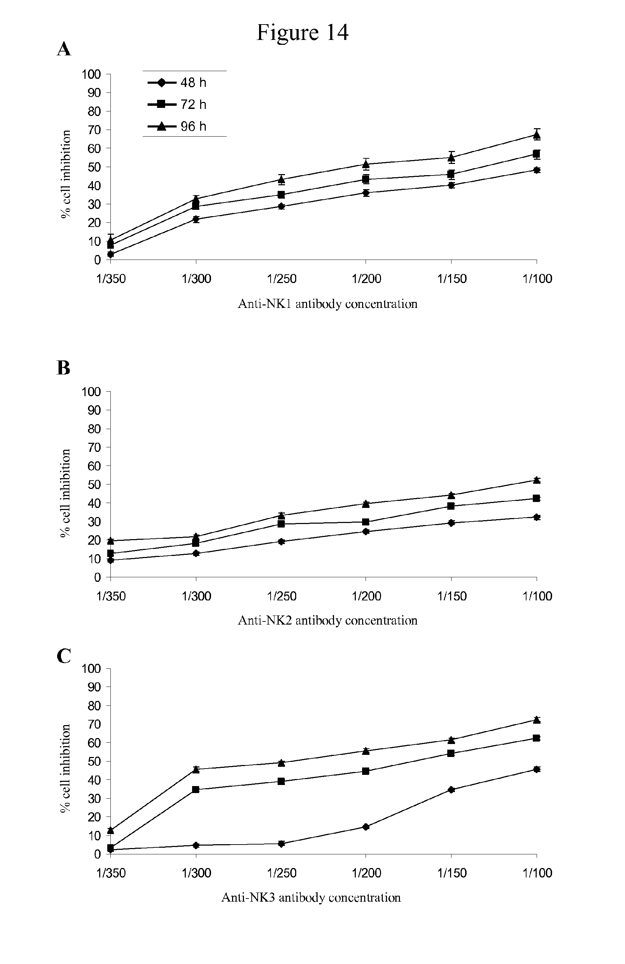

Figure 14. Inhibition of proliferation (expressed as percentage with respect to control cultures) in the commercial cell line of human carcinoma of the ovary (FU-OV-1) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

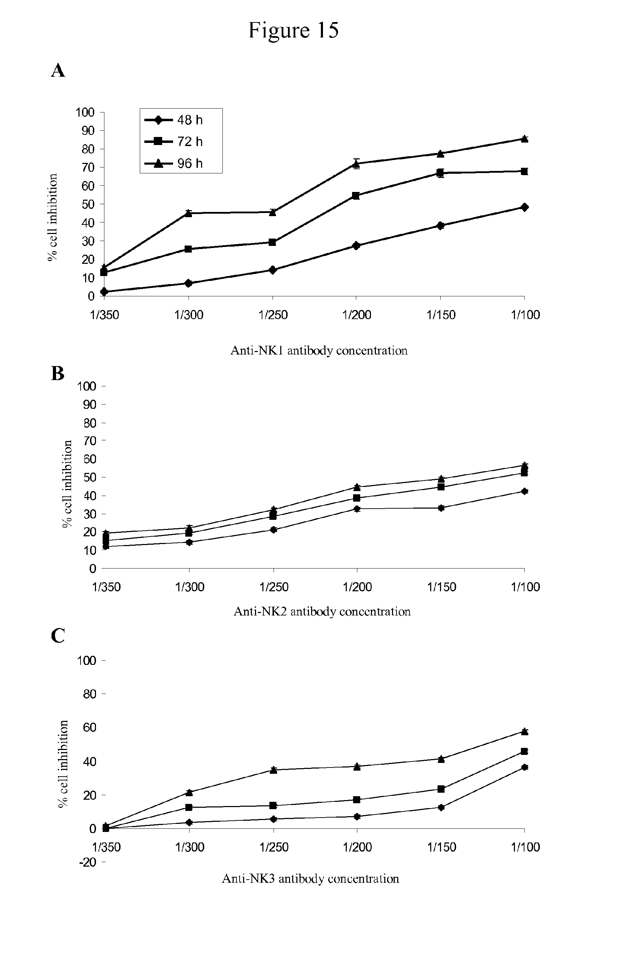

Figure 15. Inhibition of proliferation (expressed as percentage with respect to control cultures) in the commercial cell line of human carcinoma of the prostate (22RV1) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

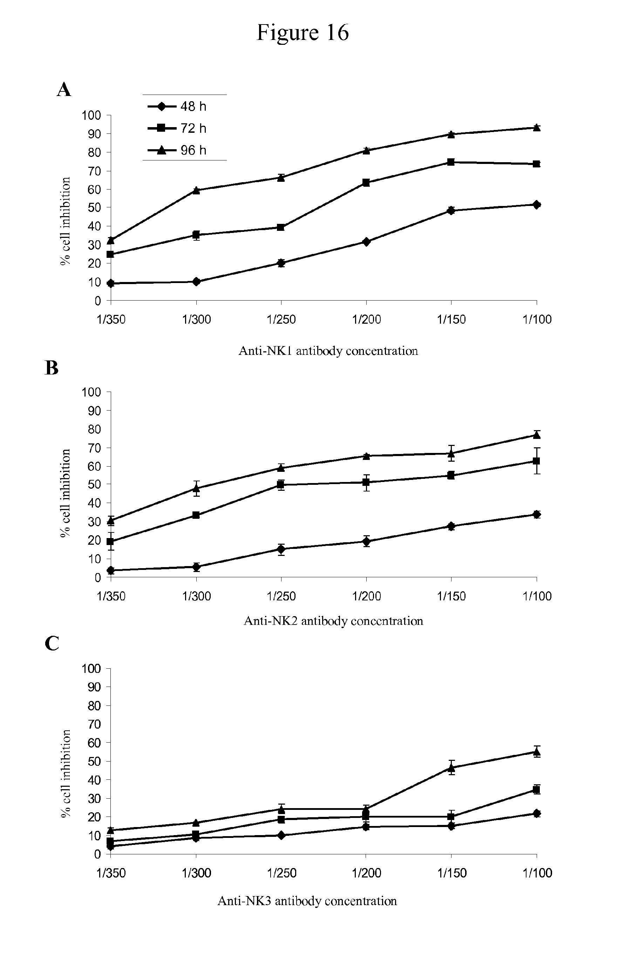

Figure 16. Inhibition of proliferation (expressed as percentage with respect to control cultures) in the commercial cell line of human small-cell lung carcinoma (HCC-44) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

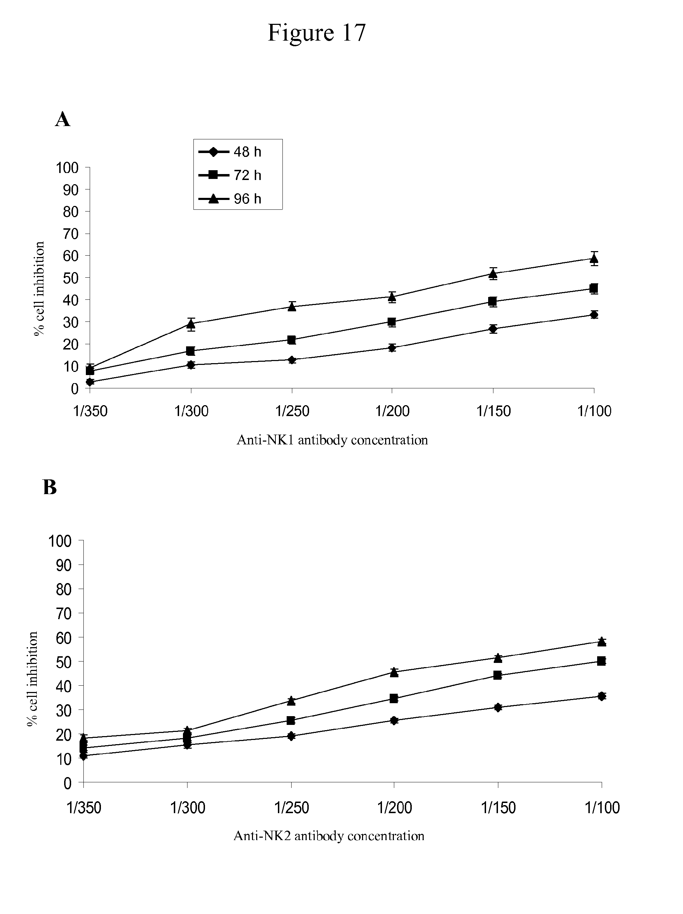

Figure 17. Inhibition of proliferation (expressed as percentage with respect to control cultures) in the commercial cell line of human non-small cell lung carcinoma (COR-L23) obtained from the collection of cell lines of the company ECACC (European Collection of Cell Cultures) and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

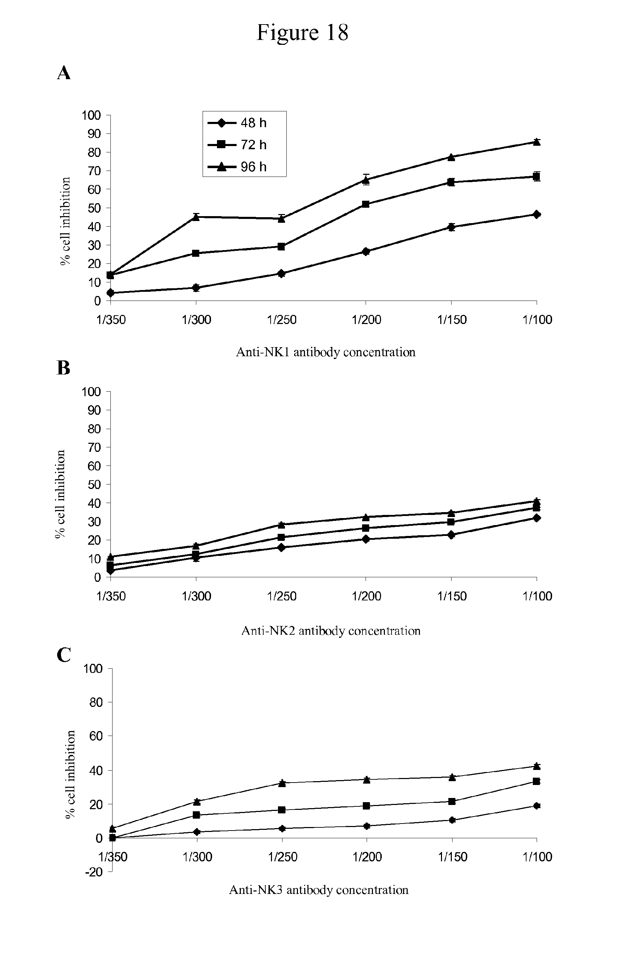

Figure 18. Inhibition of proliferation (expressed as percentage with respect to control cultures) in the commercial cell line of human small-cell lung carcinoma (H-209) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

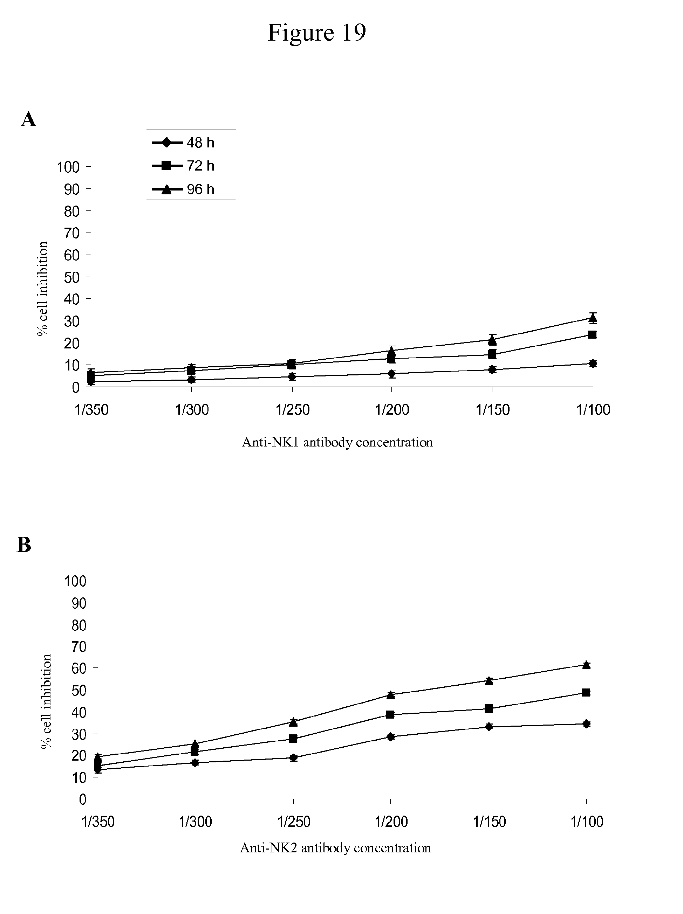

Figure 19. Inhibition of proliferation (expressed as percentage with respect to control cultures) in the commercial cell line of human small-cell lung carcinoma (H-1963) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

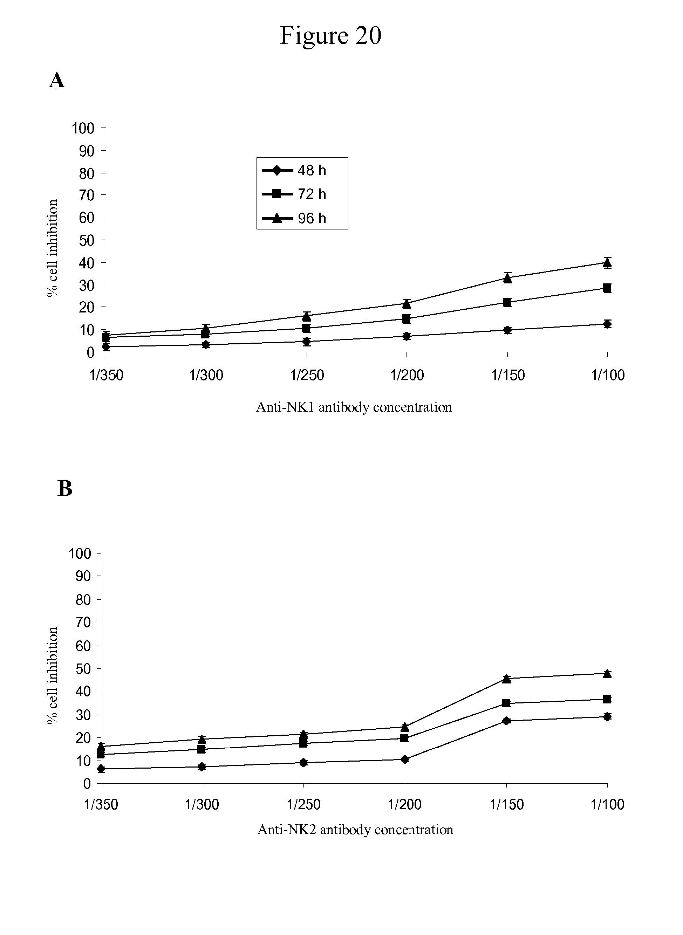

Figure 20. Inhibition of proliferation (expressed as percentage with respect to control cultures) in the commercial cell line of human small-cell lung carcinoma (H-69) obtained from the collection of cell lines of the company ECACC and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

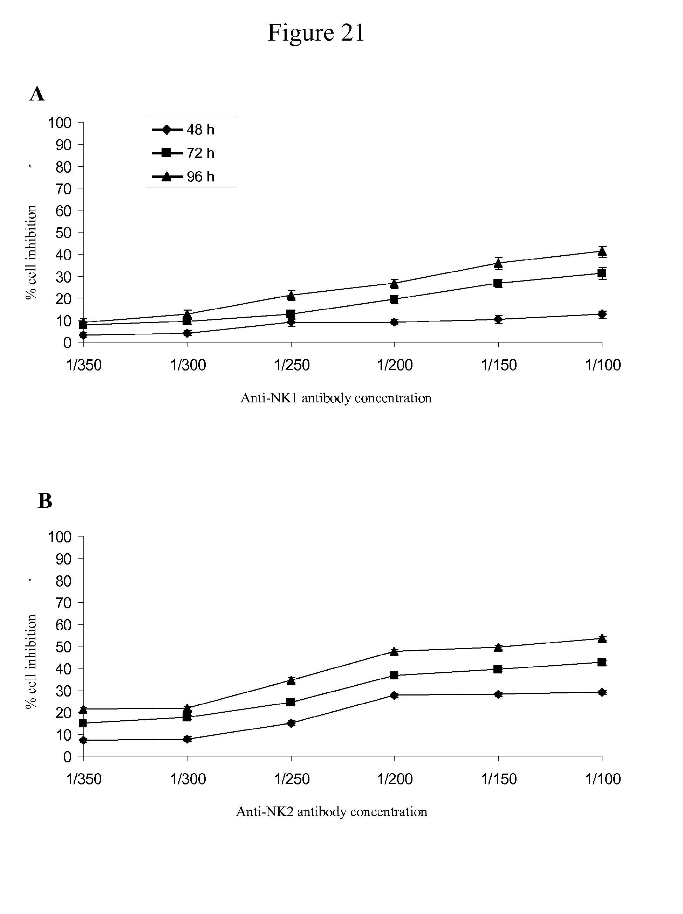

Figure 21. Inhibition of proliferation (expressed as percentage with respect to control cultures) in the commercial cell line of human lung carcinoma (A-427) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

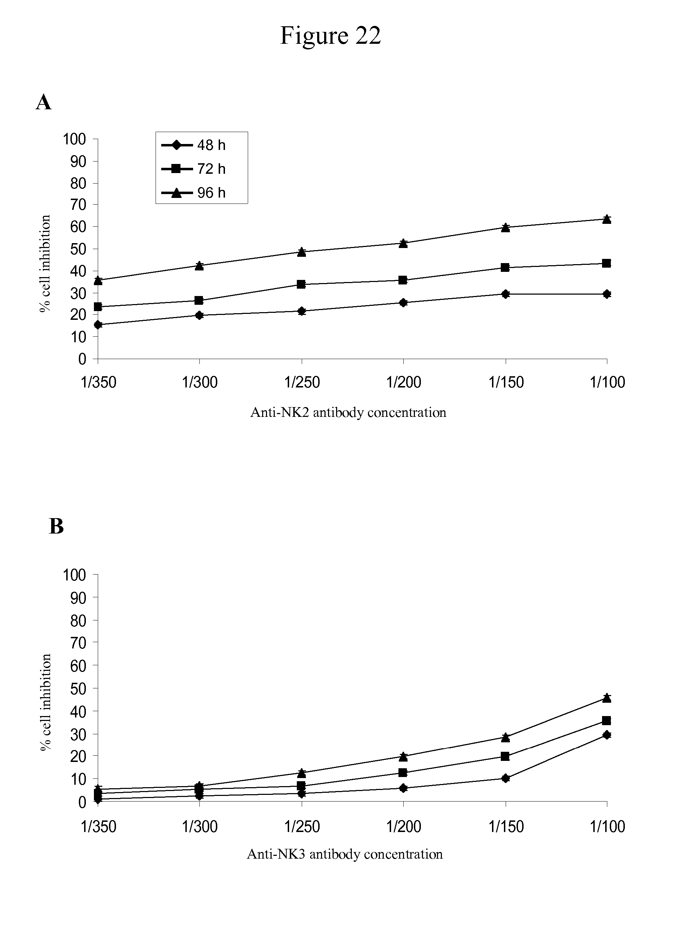

Figure 22. Inhibition of proliferation (expressed as percentage with respect to control cultures) in the commercial cell line of human thyroid carcinoma (8505C) obtained from the collection of cell lines of the company DSMZ and cultured under the conditions set out by this company, by treatment with anti-NK2 (A) or anti-NK3 (B) antibodies to antibody titres shown in the graph and for 48, 72 and 96 hours. As control of proliferation, the cells were cultured in the absence of the aforementioned antibodies.

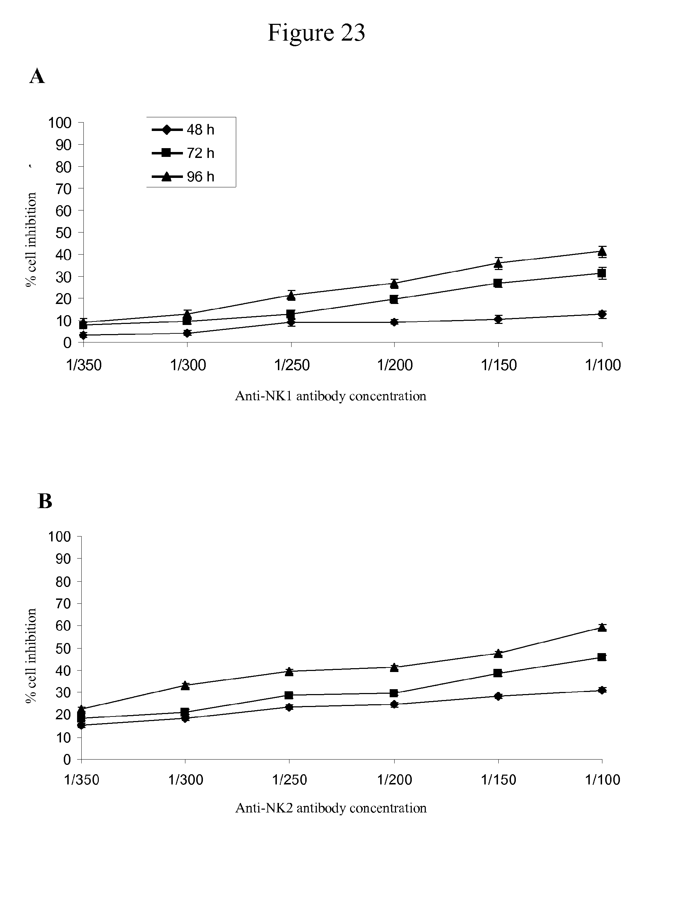

Figure 23. Inhibition of proliferation (expressed as percentage with respect to control cultures) in the commercial cell line of metastasizing human papillary thyroid carcinoma (B-CPAP) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

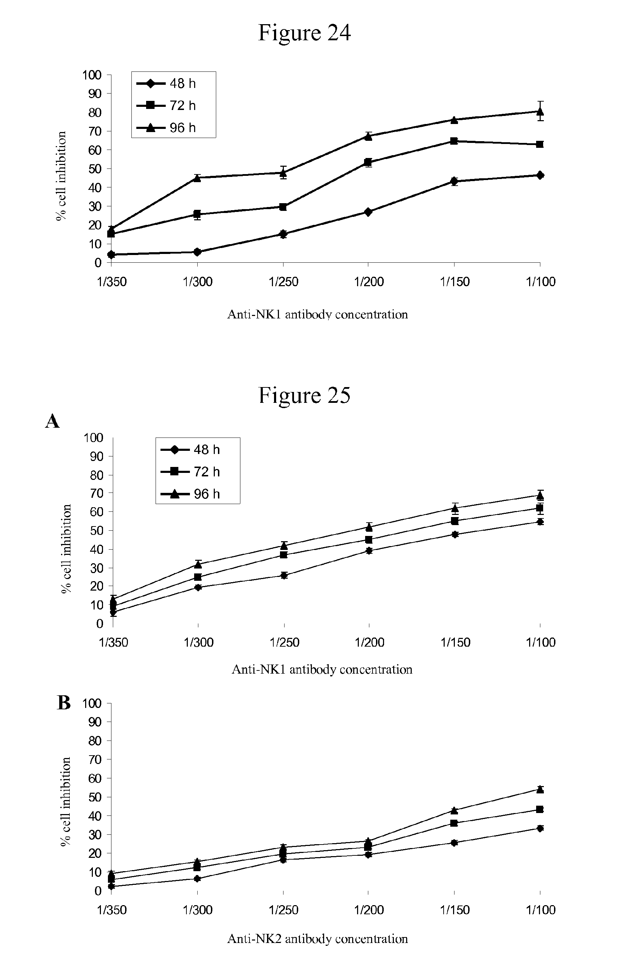

Figure 24. Inhibition of proliferation (expressed as percentage with respect to control cultures) in the commercial cell line of human carcinoma of the bladder (5637) obtained from the collection of cell lines of the company DSMZ and cultured under the conditions set out by this company, by treatment with anti-NK1 antibodies to antibody titres shown in the graph and for 48, 72 and 96 hours. As control of proliferation, the cells were cultured in the absence of the aforementioned antibodies.
Figure 25. Inhibition of proliferation (expressed as percentage with respect to control cultures) in the commercial cell line of human ductal carcinoma of the breast (BT-474) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

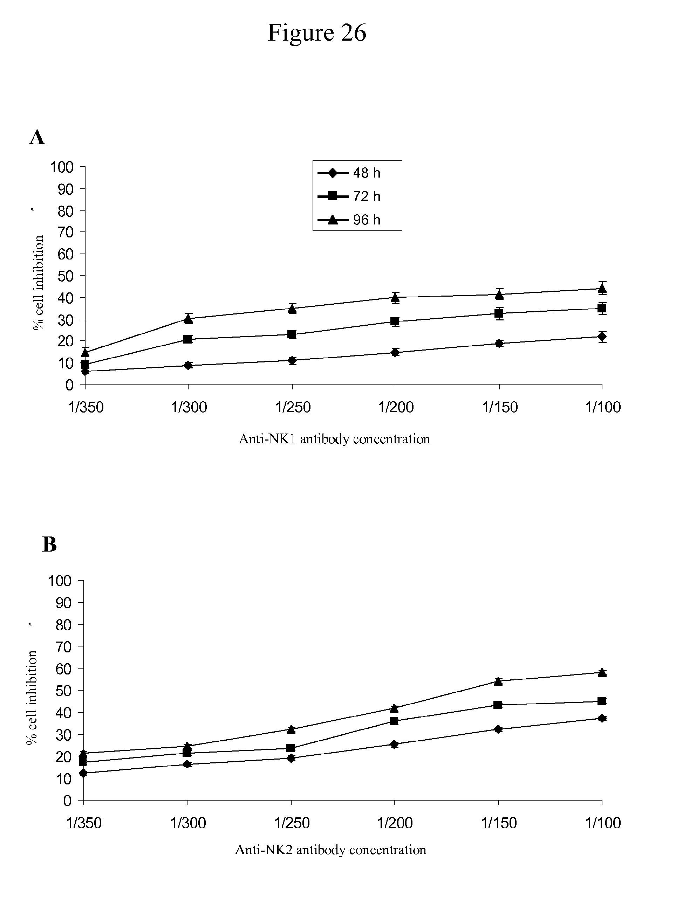

Figure 26. Inhibition of proliferation (expressed as percentage with respect to control cultures) in the commercial cell line of human follicular thyroid carcinoma (TT2609-C02) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

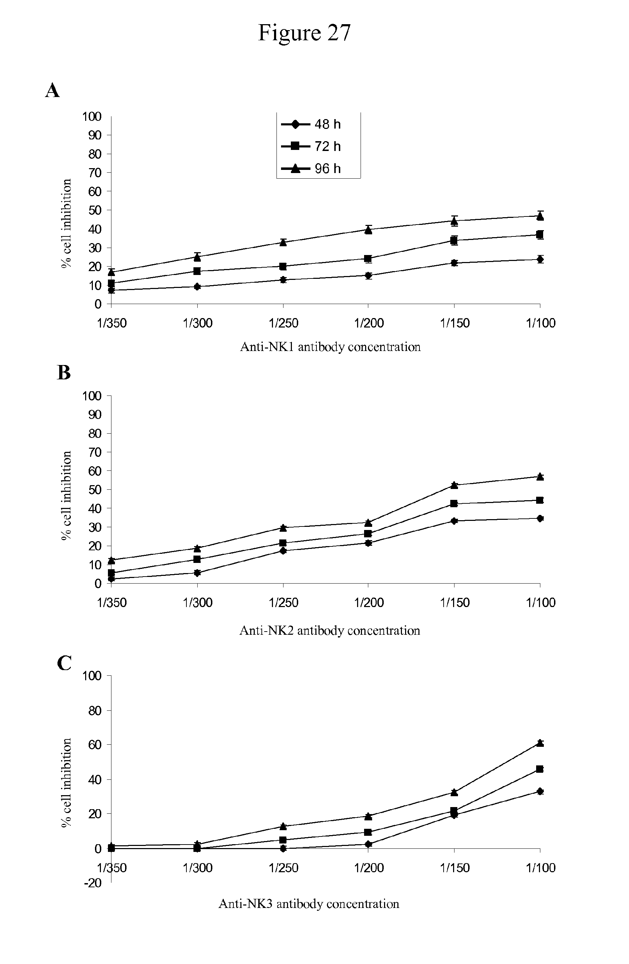

Figure 27. Inhibition of proliferation (expressed as percentage with respect to control cultures) in the commercial cell line of human transitional carcinoma of the bladder (RT-4) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

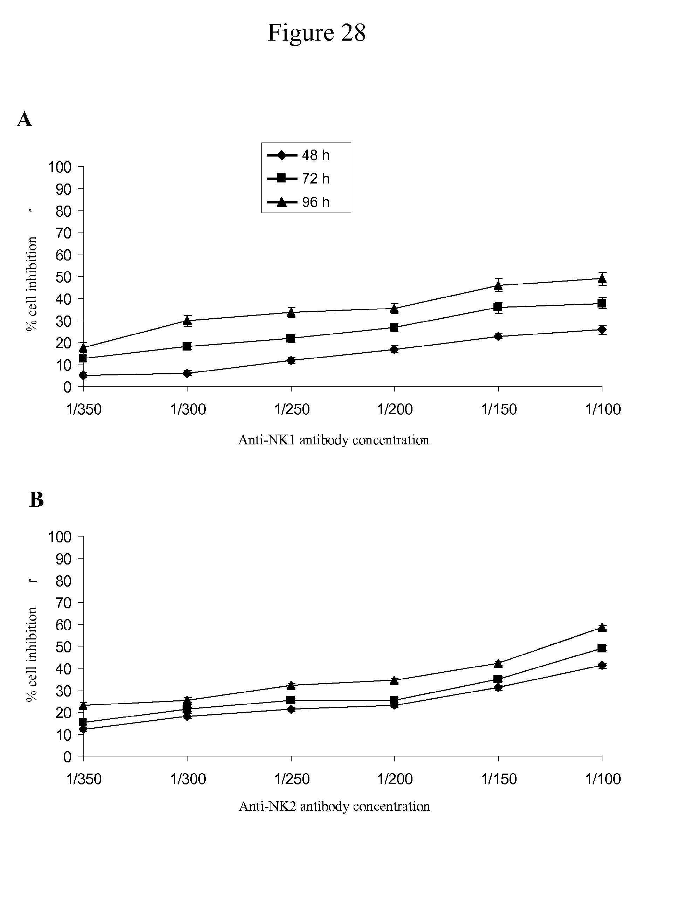

Figure 28. Inhibition of proliferation (expressed as percentage with respect to control cultures) in the commercial cell line of human urothelial bladder carcinoma (647-V) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

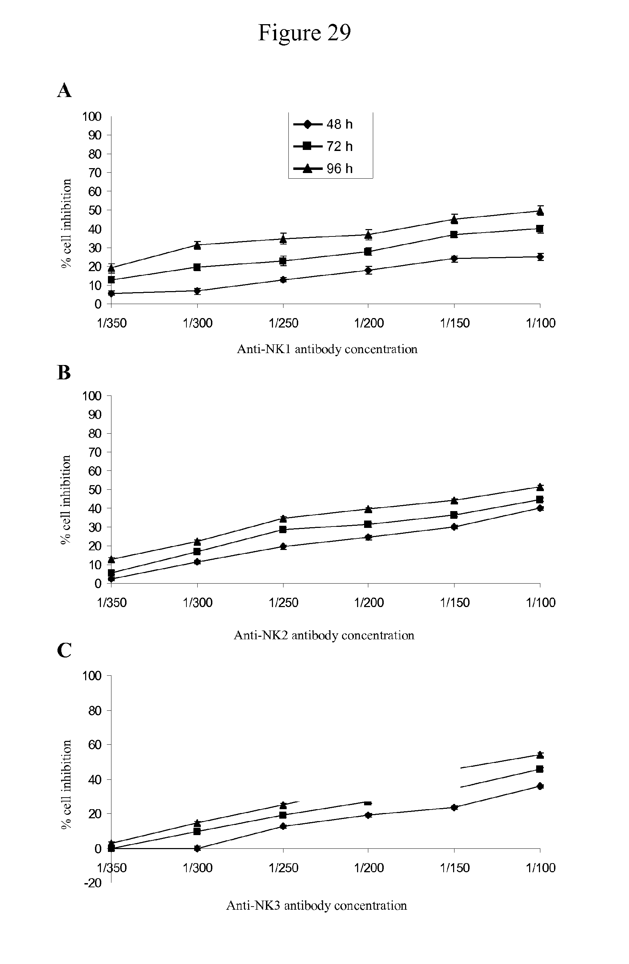

Figure 29. Inhibition of proliferation (expressed as percentage with respect to control cultures) in the commercial cell line of human choriocarcinoma (AC-1M32) obtained from the collection of cell lines of the company DSMZ and cultured under the conditions set out by this company, by the treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

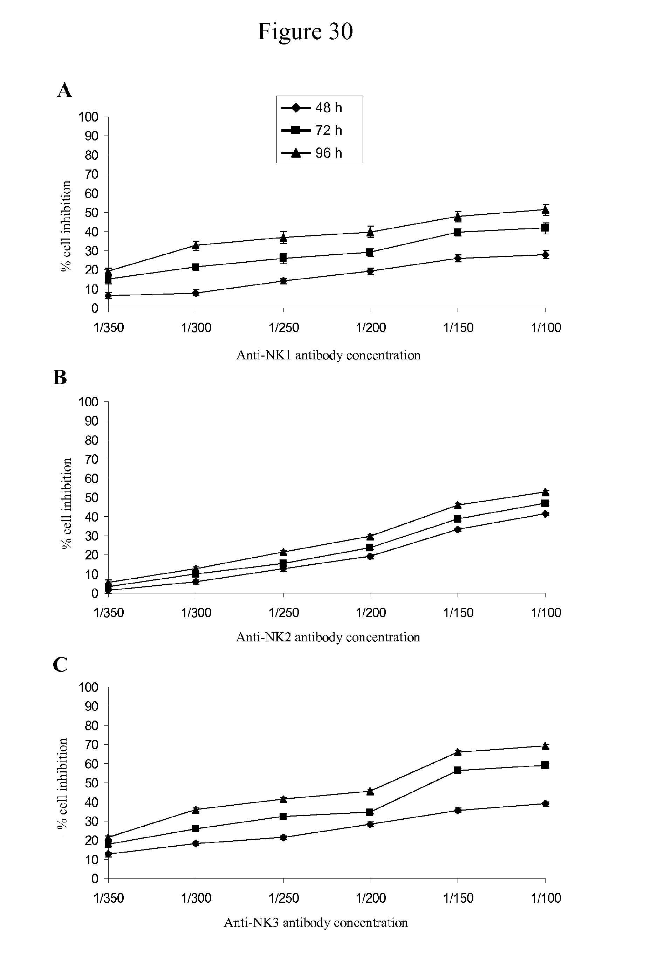

Figure 30. Inhibition of proliferation (expressed as percentage with respect to control cultures) in the commercial cell line of human fibrosarcoma (HT-1080) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

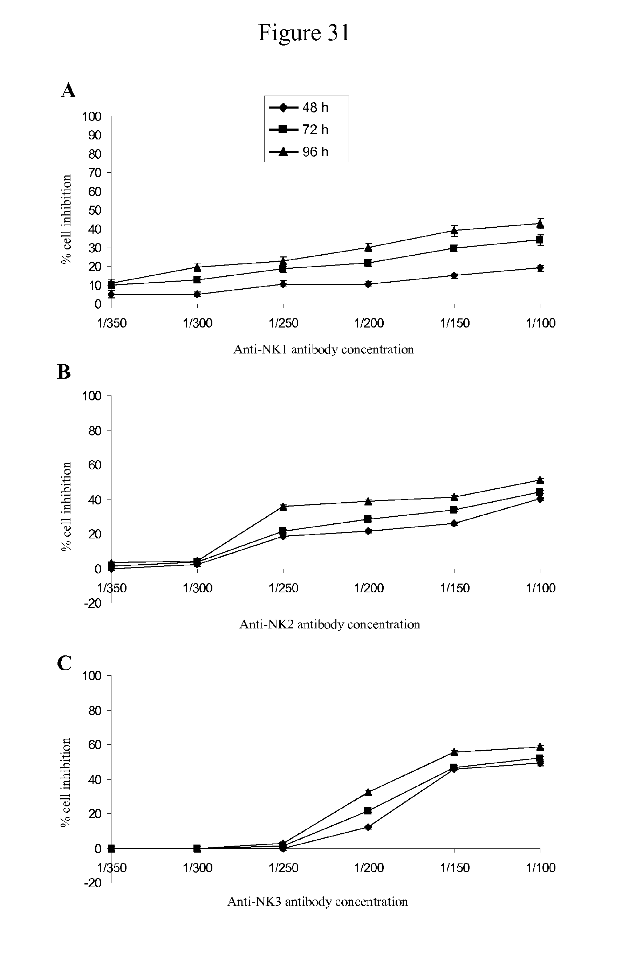

Figure 31. Inhibition of proliferation (expressed as percentage with respect to control cultures) in the commercial cell line of human glioma (GAMG) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

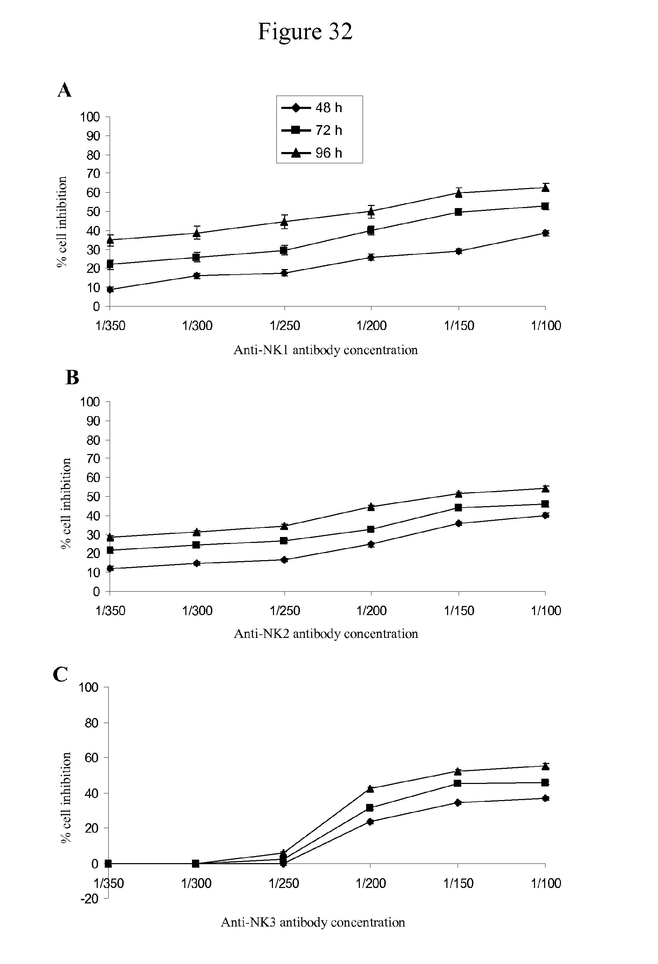

Figure 32. Inhibition of proliferation (expressed as percentage with respect to control cultures) in the commercial cell line of human malignant fibrous histiocytoma (U-2197) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

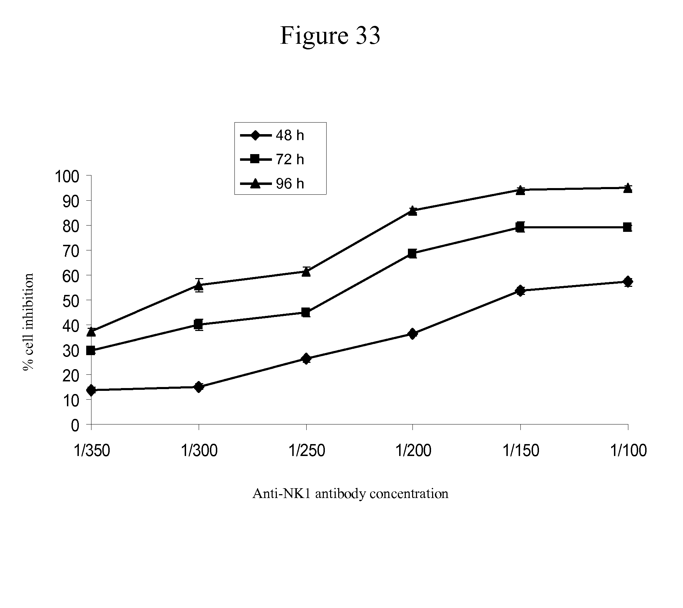

Figure 33. Inhibition of proliferation (expressed as percentage with respect to control cultures) in the commercial cell line of human T/NK cell leukaemia (YT) obtained from the collection of cell lines of the company DSMZ and cultured under the conditions set out by this company, by treatment with anti-NK1 antibodies to antibody titres shown in the graph and for 48, 72 and 96 hours. As control of proliferation, the cells were cultured in the absence of the aforementioned antibodies.

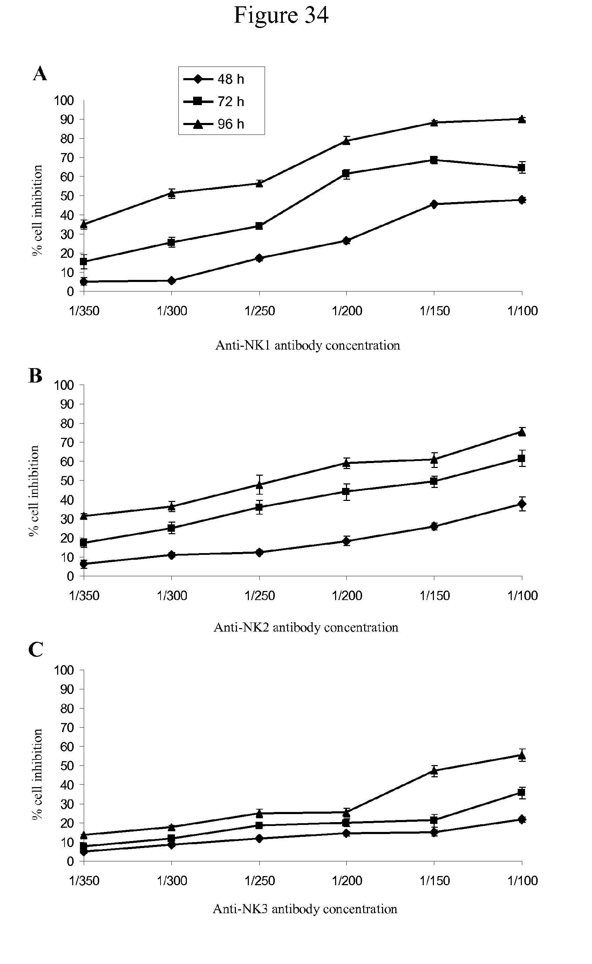

Figure 34. Inhibition of proliferation (expressed as percentage with respect to control cultures) in the commercial cell line of human B lymphoblastic leukaemia (SD1) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

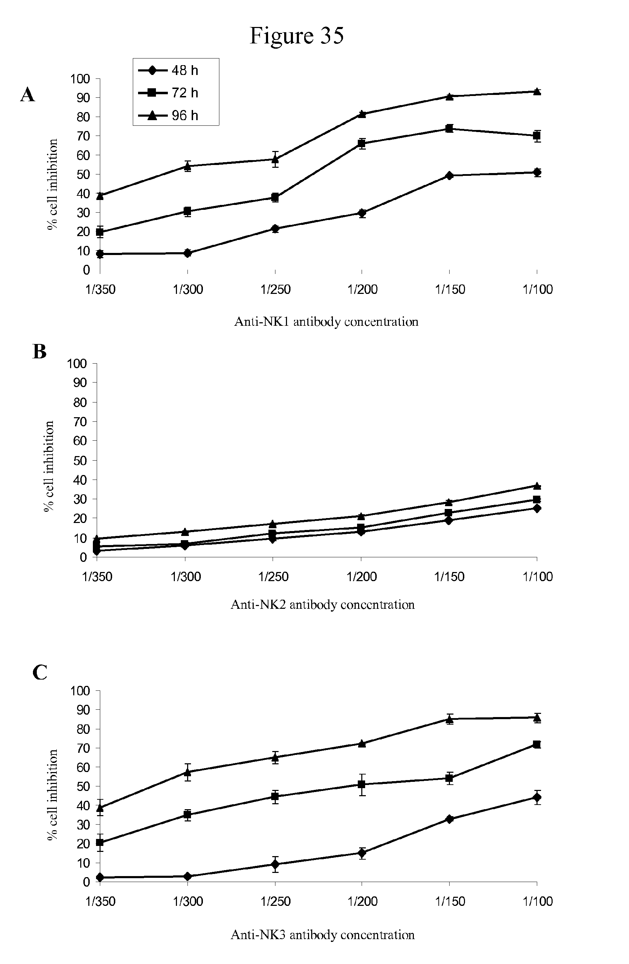

Figure 35. Inhibition of proliferation (expressed as percentage with respect to control cultures) in the commercial cell line of human T lymphoblastic leukaemia (BE-13) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

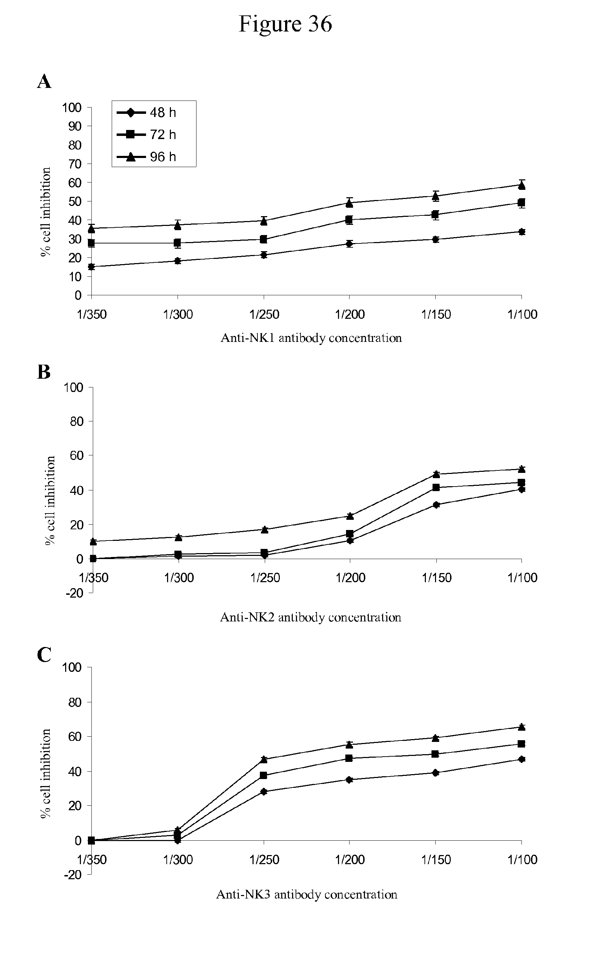

Figure 36. Inhibition of proliferation (expressed as percentage with respect to control cultures) in the commercial cell line of human B lymphoma (BC-1) obtained from the collection of cell lines of the company DSMZ and cultured under the conditions set out by this company, by the treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

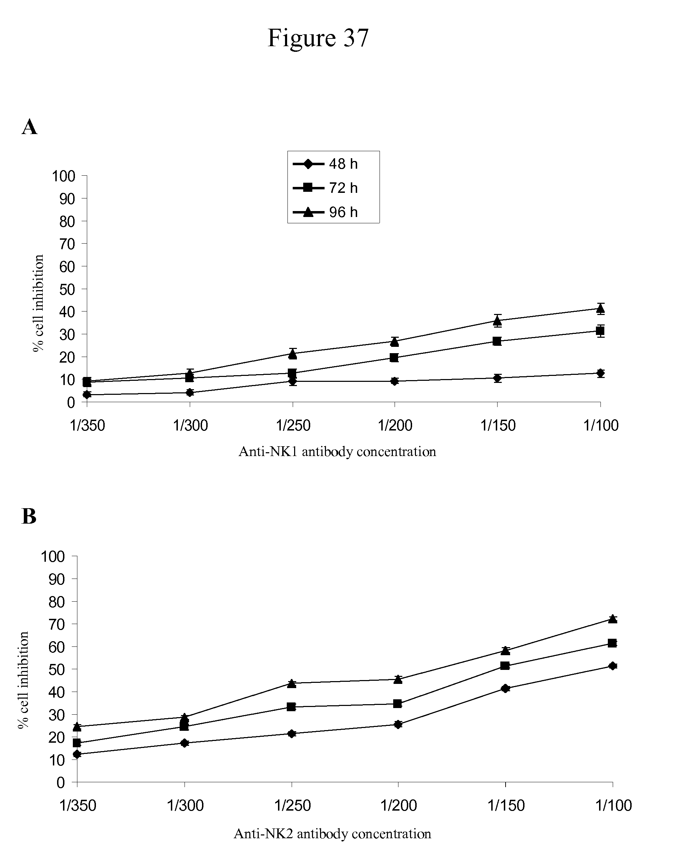

Figure 37. Inhibition of proliferation (expressed as percentage with respect to control cultures) in the commercial cell line of human B lymphoma (SU-DHL-16) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

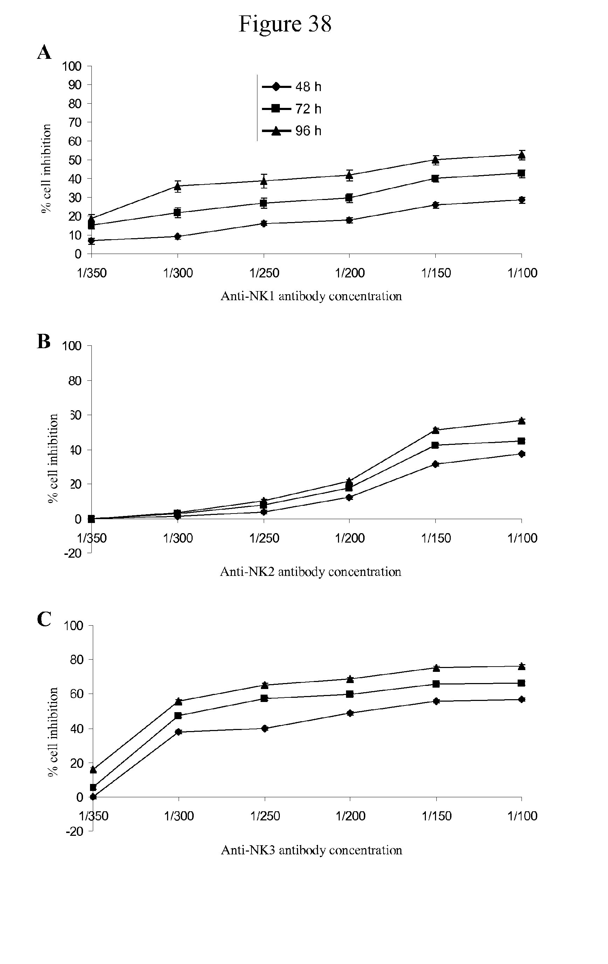

Figure 38. Inhibition of proliferation (expressed as percentage with respect to control cultures) in the commercial cell line of human Burkitt lymphoma (CA-46) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

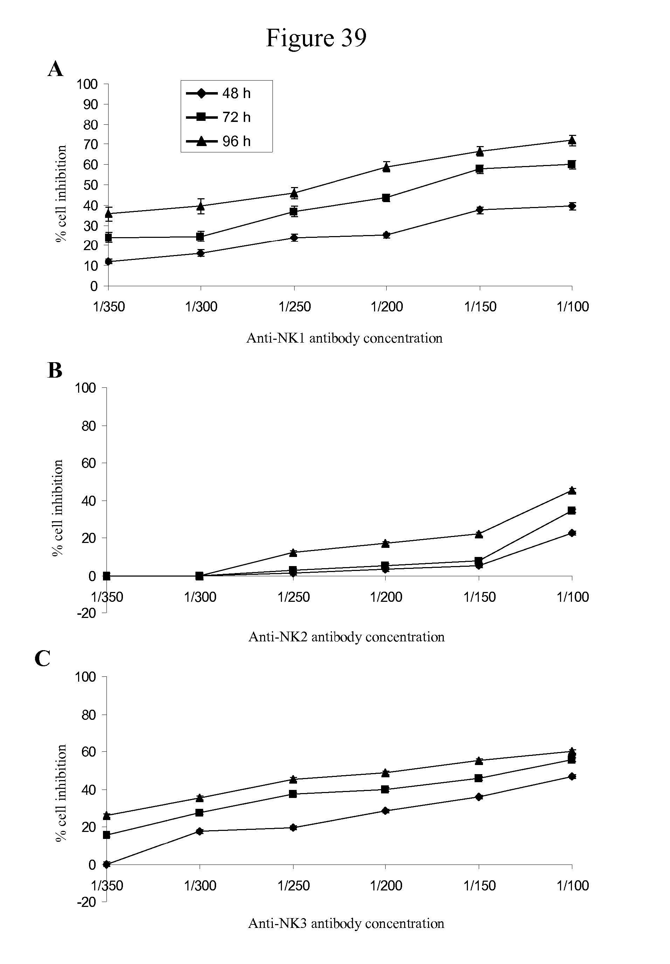

Figure 39. Inhibition of proliferation (expressed as percentage with respect to control cultures) in the commercial cell line of human Hodgkin lymphoma (KM-H2) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

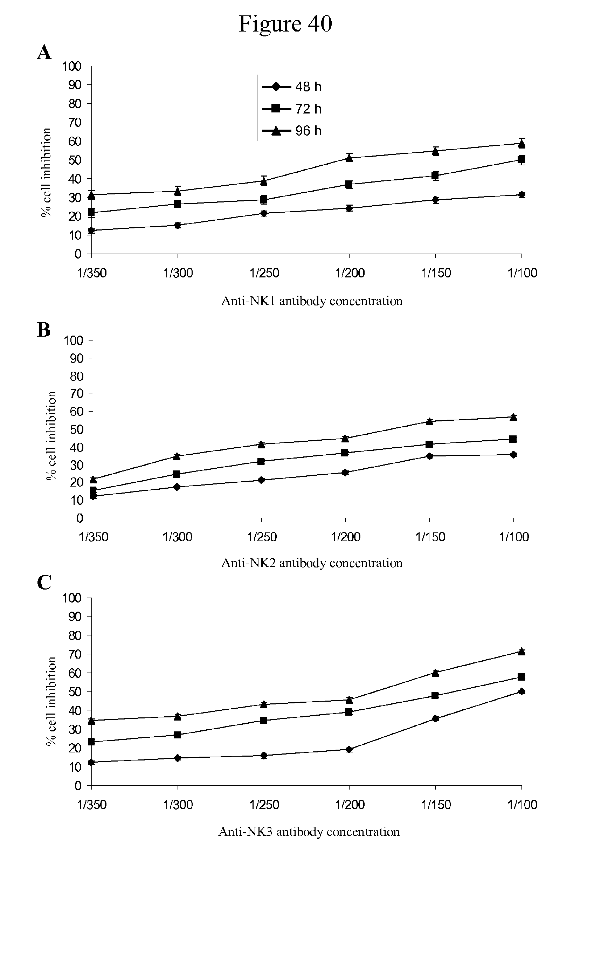

Figure 40. Inhibition of proliferation (expressed as percentage with respect to control cultures) in the commercial cell line of human T lymphoma (DERL-7) obtained from the collection of cell lines of the company DSMZ and cultured under the conditions set out by this company, by the treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

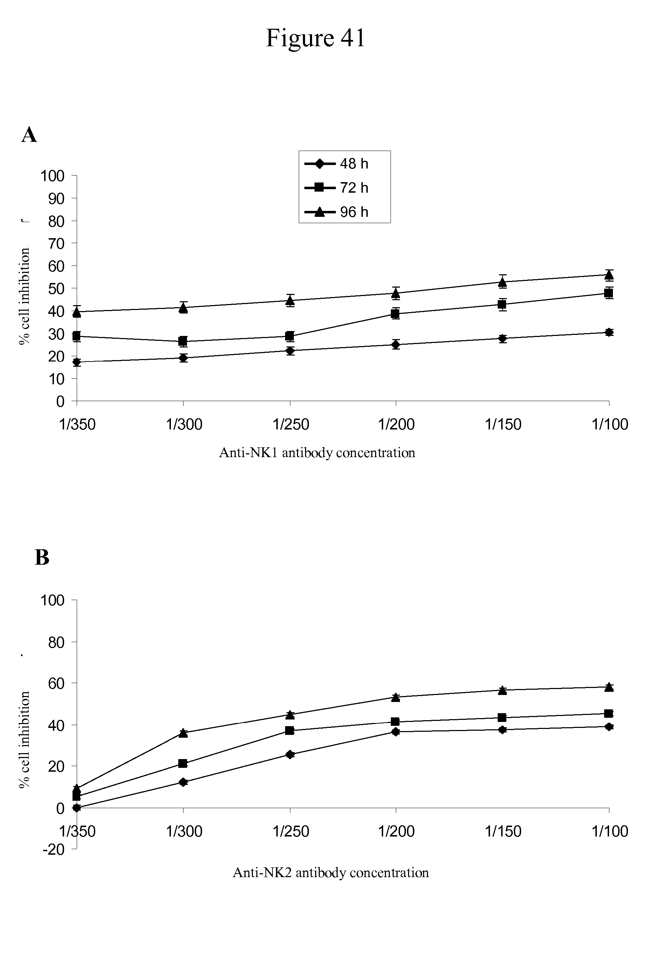

Figure 41. Inhibition of proliferation (expressed as percentage with respect to control cultures) in the commercial cell line of human T lymphoma (SUP-T1) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

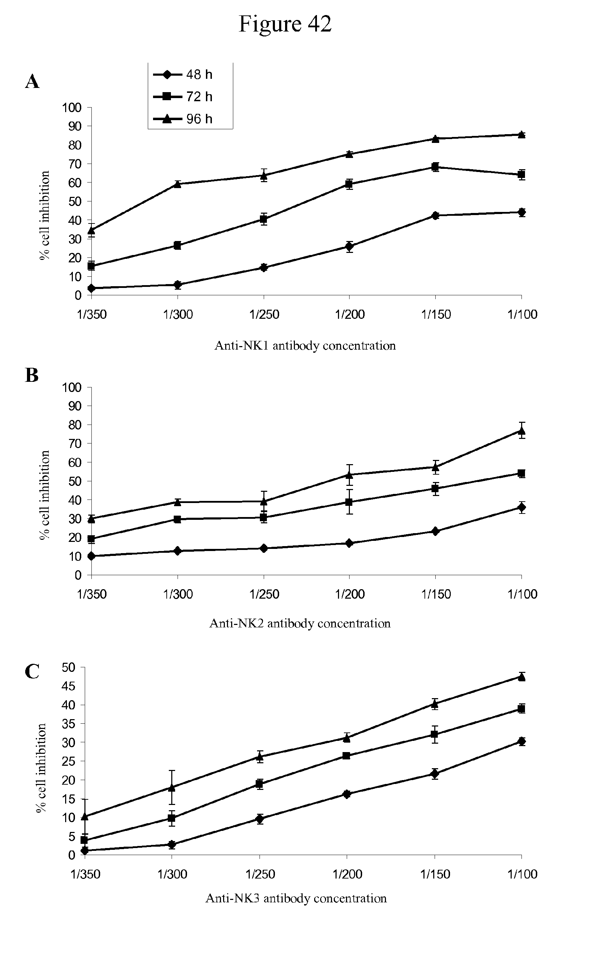

Figure 42. Inhibition of proliferation (expressed as percentage with respect to control cultures) in the commercial cell line of human melanoma (MEL HO) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

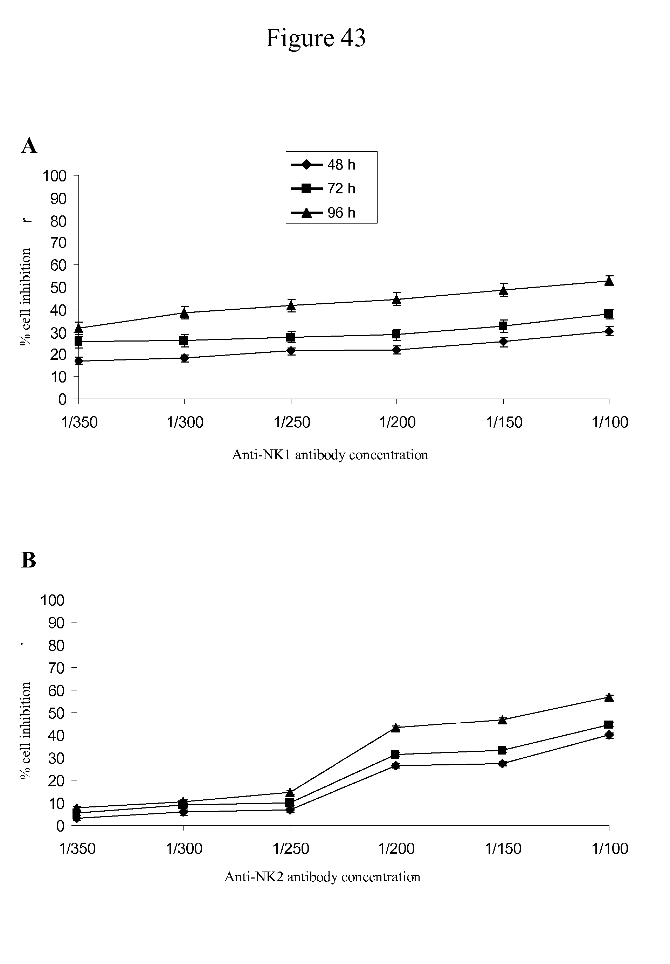

Figure 43. Inhibition of proliferation (expressed as percentage with respect to control cultures) in the commercial cell line of human melanoma (COLO 679) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

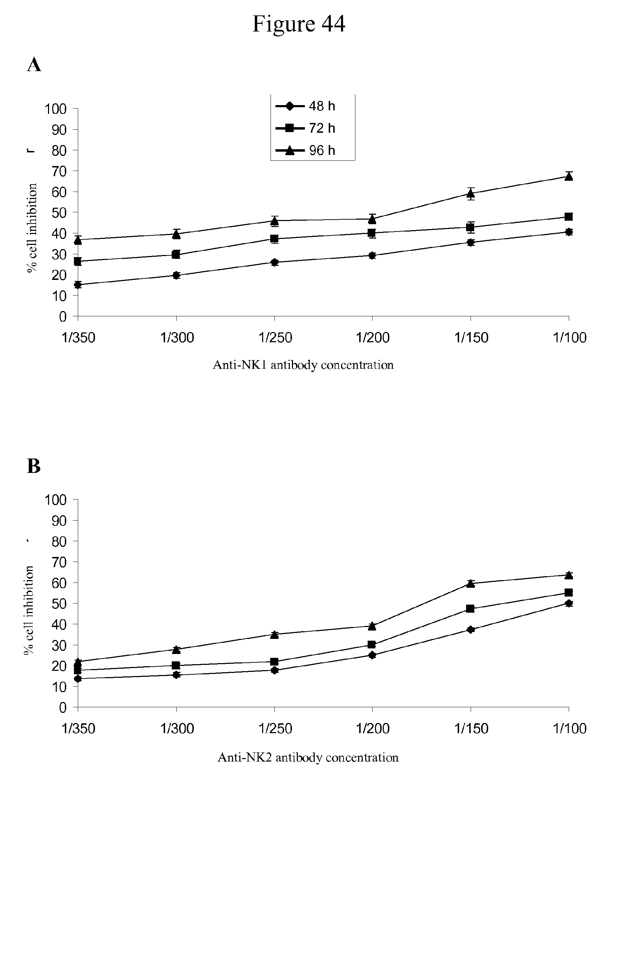

Figure 44. Inhibition of proliferation (expressed as percentage with respect to control cultures) in the commercial cell line of human melanoma with metastasis in lymph node (COLO 858) obtained from the collection of cell lines of the company ICLC (Interlab Cell line collection -CBA- Genoa) and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

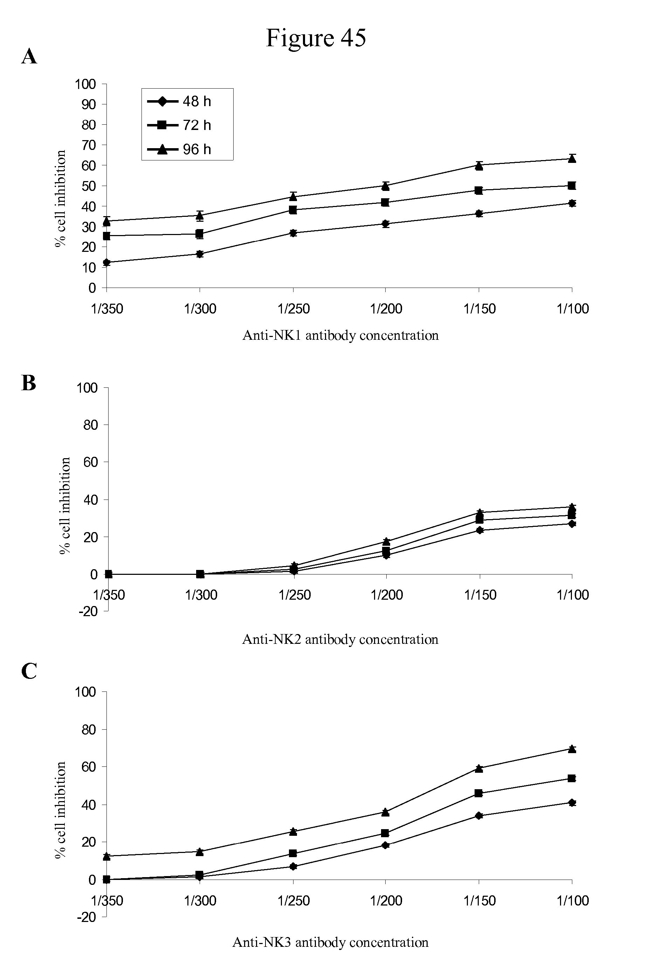

Figure 45. Inhibition of proliferation (expressed as percentage with respect to control cultures) in the commercial cell line of human multiple myeloma (COLO 677) obtained from the collection of cell lines of the company DSMZ and cultured under the conditions set out by this company, by the treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

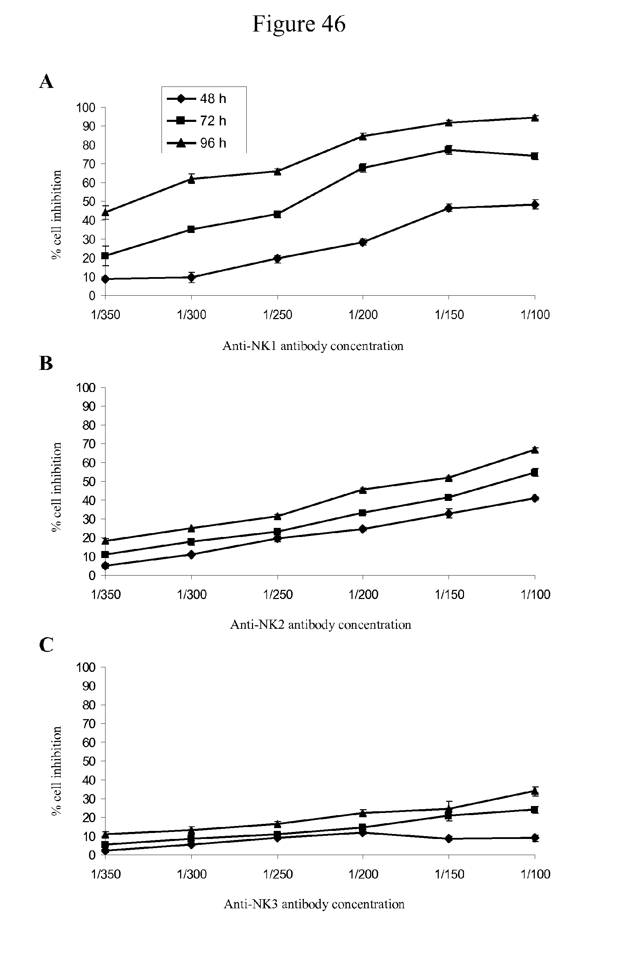

Figure 46. Inhibition of proliferation (expressed as percentage with respect to control cultures) in the commercial cell line of human neuroblastoma (KELLY) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

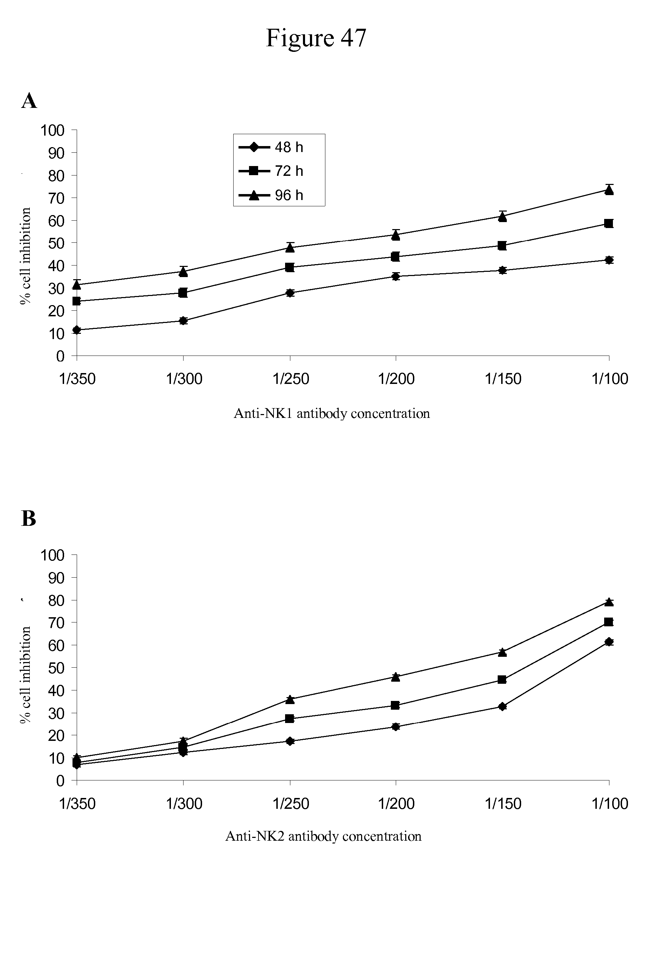

Figure 47. Inhibition of proliferation (expressed as percentage with respect to control cultures) in the commercial cell line of human neuroblastoma (IMR-32) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

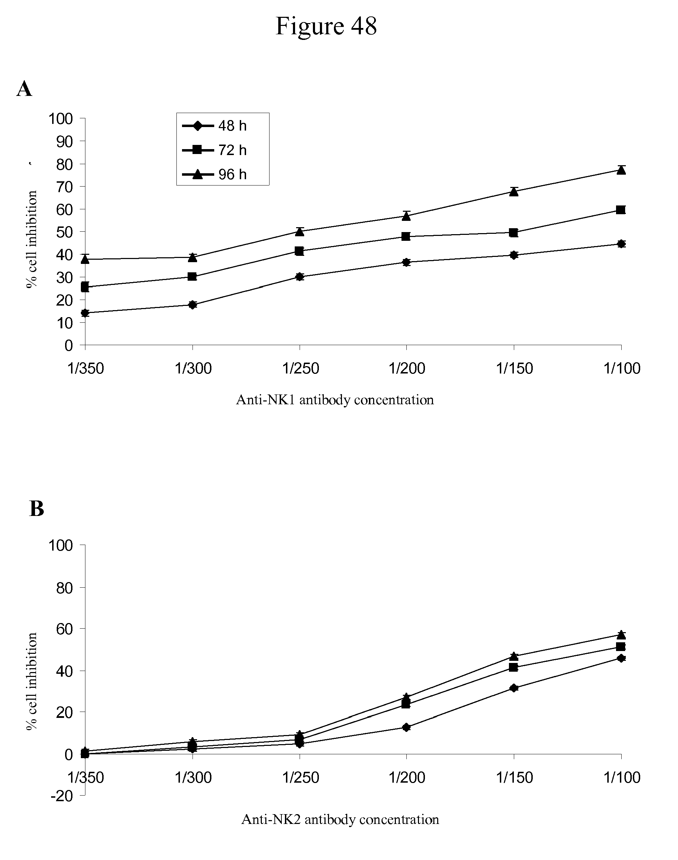

Figure 48. Inhibition of proliferation (expressed as percentage with respect to control cultures) in the commercial cell line of human neuroblastoma, bone marrow (SKN-BE 2) obtained from the collection of cell lines of the company ICLC and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

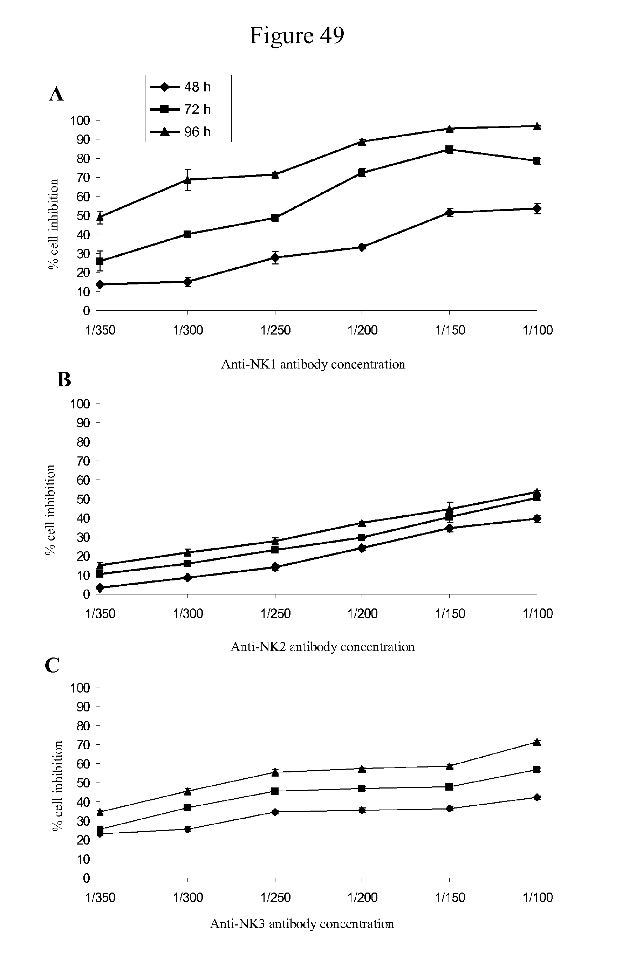

Figure 49. Inhibition of proliferation (expressed as percentage with respect to control cultures) in the commercial cell line of human osteosarcoma (MG-63) obtained from the collection of cell lines of the company ICLC and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

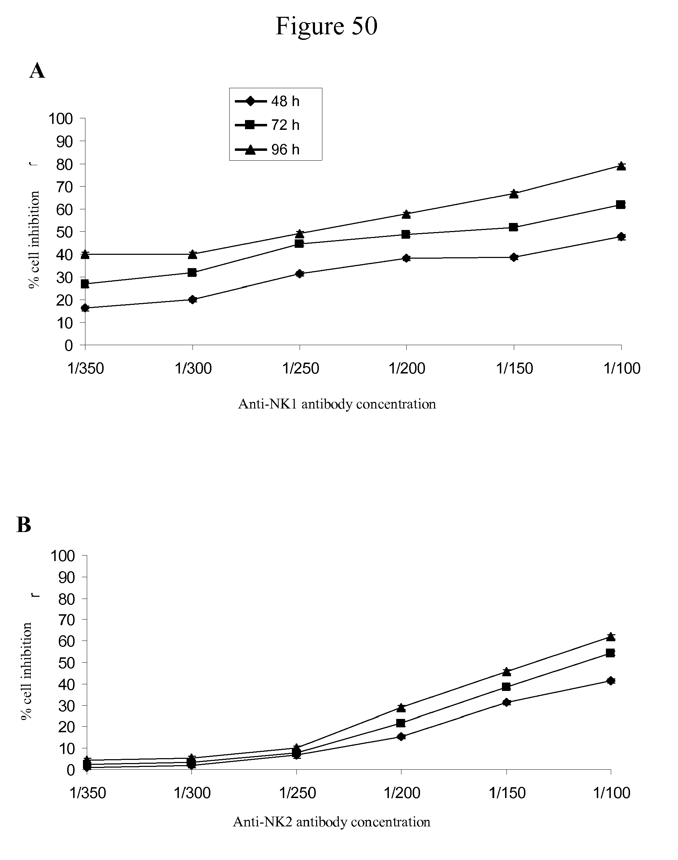

Figure 50. Inhibition of proliferation (expressed as percentage with respect to control cultures) in the commercial cell line of human osteosarcoma (CAL-72) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

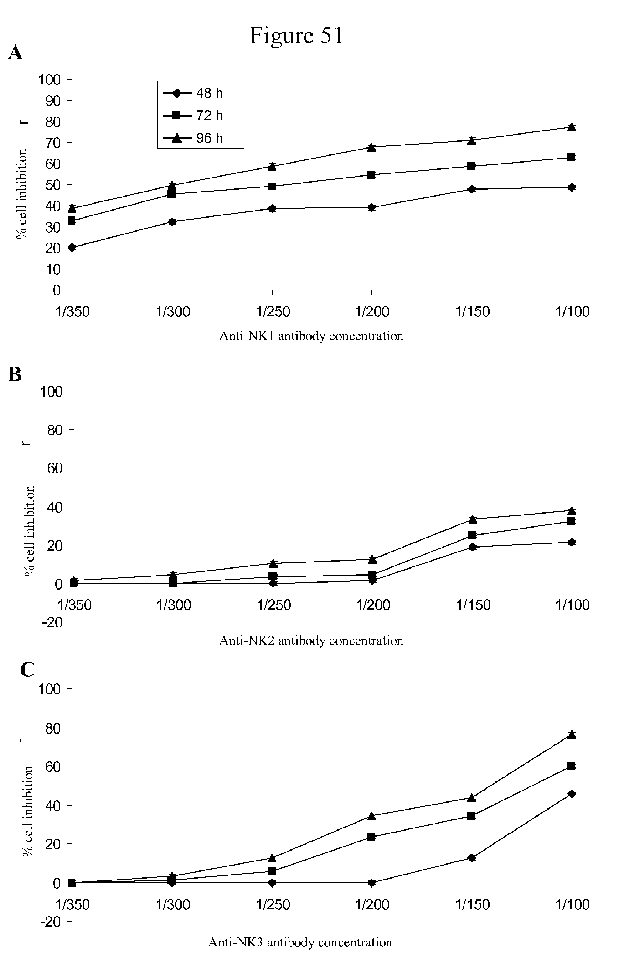

Figure 51. Inhibition of proliferation (expressed as percentage with respect to control cultures) in the commercial cell line of human rhabdomyosarcoma (A-204) obtained from the collection of cell lines of the company DSMZ and cultured under the conditions set out by this company, by the treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

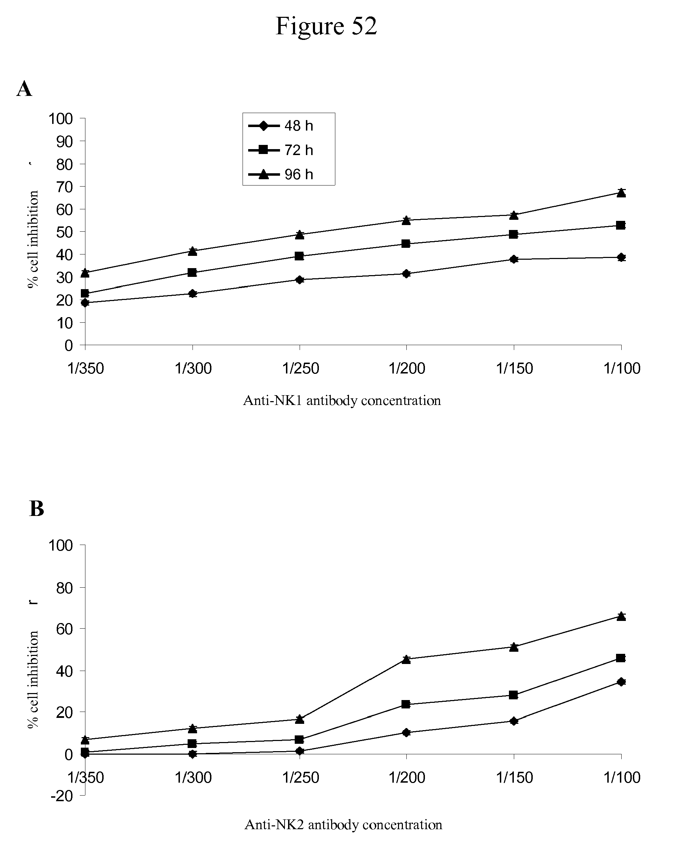

Figure 52. Inhibition of proliferation (expressed as percentage with respect to control cultures) in the commercial cell line of human retinoblastoma (Y-79) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

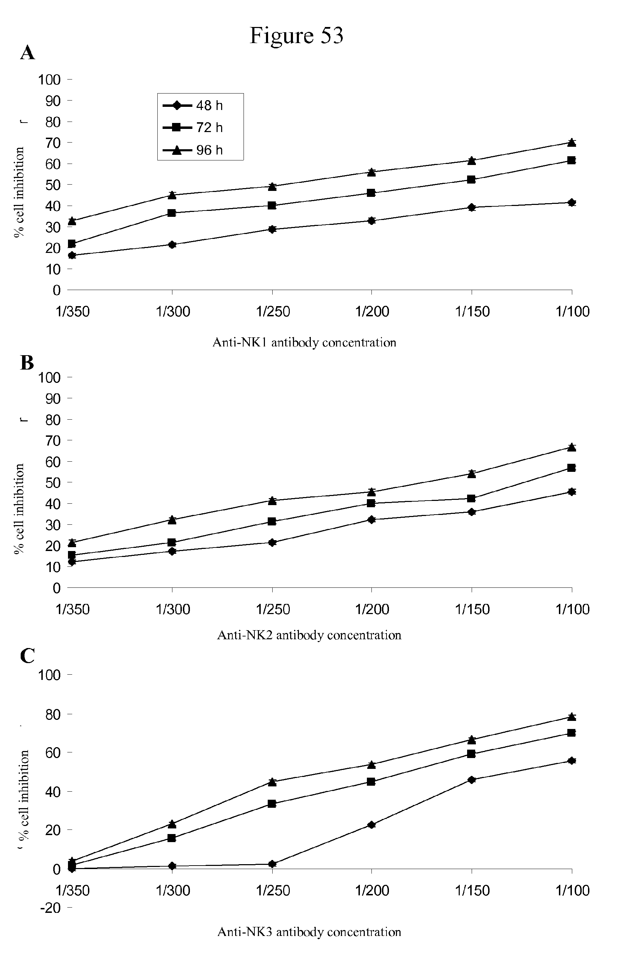

Figure 53. Inhibition of proliferation (expressed as percentage with respect to control cultures) in the commercial cell line of human retinoblastoma (WERI-Rb-1) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

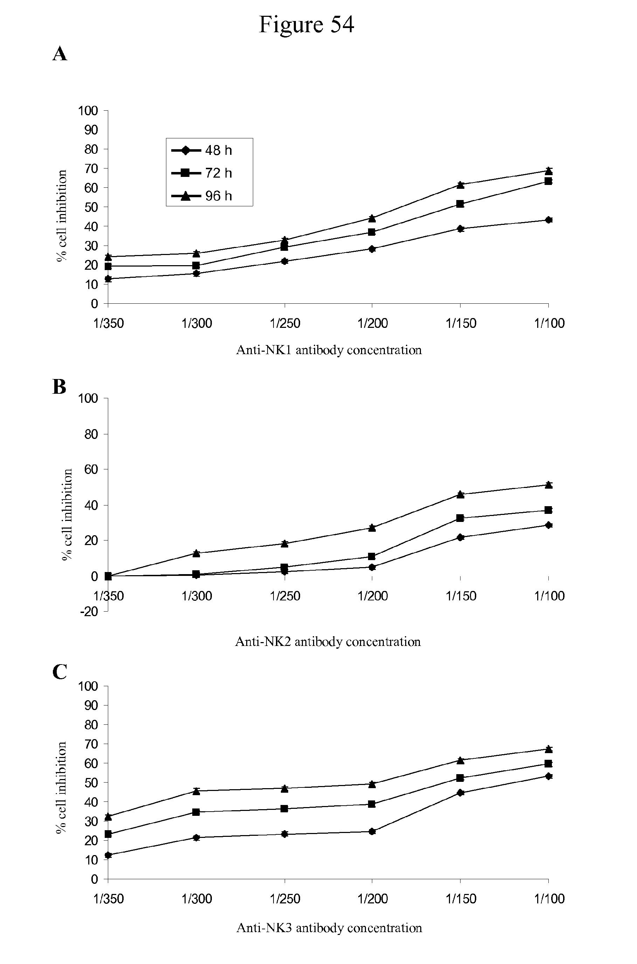

Figure 54. Inhibition of proliferation (expressed as percentage with respect to control cultures) in the commercial cell line of human Edwing sarcoma (MHH-ES-1) obtained from the collection of cell lines of the company DSMZ and cultured under the conditions set out by this company, by treatment with anti-NK1 (A), anti-NK2 (B) or anti-NK3 (C) antibodies to antibody titres shown in the graph and for 48, 72 and 96 hours. As control of proliferation, the cells were cultured in the absence of the aforementioned antibodies.

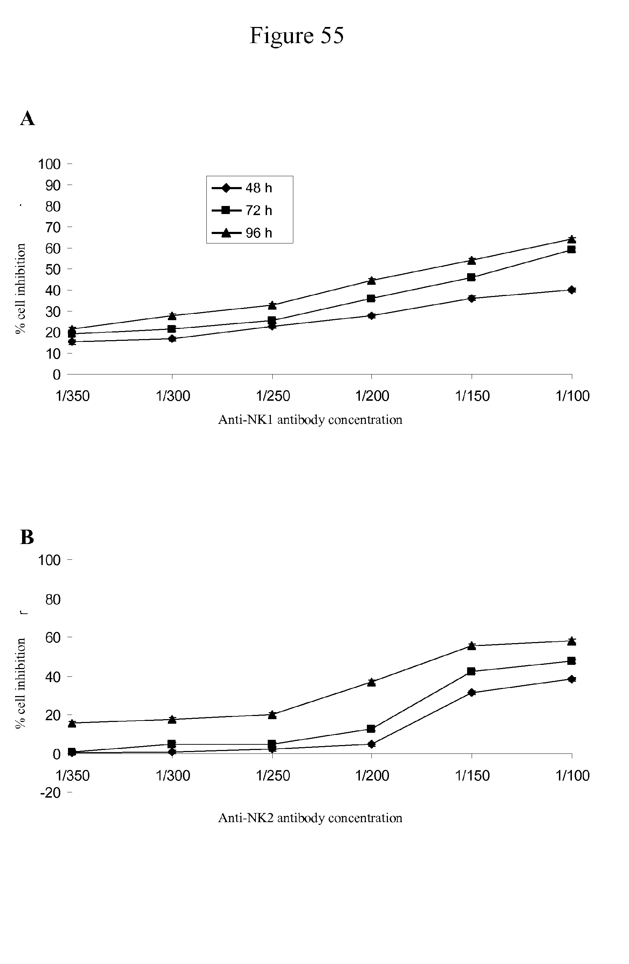

Figure 55. Inhibition of proliferation (expressed as percentage with respect to control cultures) in the commercial cell line of human endometrial stromal sarcoma (ESS-1) obtained from the collection of cell lines of the company DSMZ and cultured under the conditions set out by this company, by treatment with anti-NK1 (A) or anti-NK2 (B) antibodies to antibody titres shown in the graph and for 48, 72 and 96 hours. As control of proliferation, the cells were cultured in the absence of the aforementioned antibodies.

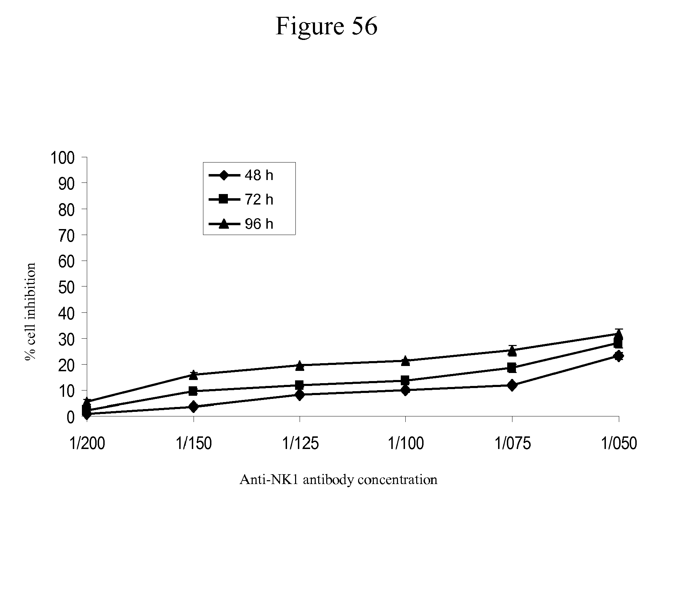

Figure 56. Inhibition of proliferation (expressed as percentage with respect to the control cultures) in the human endothelial cell line (C-12210) by the treatment with the anti-NK1 (A) antibody to the antibody titres shown in the graph and for 48, 72 and 96 hours. As control of proliferation, the cells were cultured in the absence of the aforementioned antibody.

### Detailed description of the invention

One of the embodiments of the invention refer to the use of at least one antibody or fragment thereof, specific against NK1, NK2 and/or NK3 cell receptors, or combinations thereof, for the manufacture of a medicament or a pharmaceutical composition for the treatment of cancer.

Another embodiment of the invention refers to the use of at least one antibody or fragment thereof, specific against NK1, NK2 and/or NK3 cell receptors, or combinations thereof, for the manufacture of a medicament or a pharmaceutical composition for the induction of apoptosis in tumour cells.

Another preferred embodiment of the invention comprises the use of at least one antibody or fragment thereof, specific against NK1, NK2 and/or NK3 cell receptors, or combinations thereof, for the manufacture of a medicament or a pharmaceutical composition to reduce tumour size. This tumour size reduction is performed by modifying the peritumoural microenvironment, which comprises modification of the physiology of the stromal matrix and stromal cells around the tumours, modification of the physiology of the cells of this matrix (fibroblasts), modification of the physiology of cells of the inflammatory and/or immune response, and modification of the physiology of cells of vascularisation, such as vascular endothelium cells around of the tumoural and peritumoural areas. All of this aimed to the modification of the physiology of peritumoural areas that show unique characteristics, different from the physiology of them in the absence of tumour, that determine the peritumoural microenvironment and promote tumour development and progression. The modification of this unique physiology of the peritumoural areas by the use of anti-NK1, anti-NK2 and anti NK-3 antibodies is aimed at reducing tumour size and preventing their progression. In this regard, the modification of the physiology of the stromal matrix and stromal cells and/or the physiology of the cells involved in the inflammatory and immune response comprises the inhibition of tumour markers. These tumour markers are selected preferably from any of the list: NF-kB, TGF alpha, TGF-beta 1, TGF-beta 2, TGF-beta 3, SPARC, MMP-3, MMP-7, MMP-9, MMP-11, MMP-13, MMP-14 and/or any combinations thereof. In addition, the modification of the physiology of the cells responsible for vascularisation comprises the inhibition of neovascularisation, preferably by the inhibition of vascular endothelial cell proliferation. As discussed above, all of this is aimed at reducing tumour size and preventing its progression.

The type of cancer to be treated with the use of the invention compounds is selected from the group comprising melanoma, glial tumours, carcinomas, adenocarcinomas, leukaemia, lymphomas, myelomas, neuroblastomas (and other tumours of germ cell or neuroectodermic type), and/or sarcomas.

More preferably, the carcinoma is selected from any of the following: breast, including ductal, lung, including small cells and non-small cells, thyroid, including follicular and papillary, digestive system, prostate, cervix, endometrium, ovary, bladder, including endothelial and transitional and/or choriocarcinoma.

In addition, the adenocarcinoma is selected from any of the following: breast, ovary, pancreas, lung and digestive system, including the stomach, and the colon.

On the other hand, leukaemia is preferably human T cell leukaemia, B cell leukaemia, and/or T/NK cell lymphoblastic leukaemia. Lymphomas are preferably of B type, T type, Hodgkin lymphoma and/or of Burkitt lymphoma.

The types of sarcoma to be treated by the use of the antibodies discloses in the invention are preferably osteosarcomas, rhabomyosarcomas, fibrosarcomas, malignant fibrous histiocytoma, sarcoma of the endometrial stroma and/or Edwing sarcoma.

On the other hand, other neoplasms to be treated by the use of the antibodies discloses in the invention are preferably neuroblastoma, as well as other neoplasms of germ cell and/or neuroectodermic origin.

The antibody used in this invention, preferably, recognises at least a sequence of NK1, NK2 or NK3 receptors, selected among: SEQ ID NO:1, SEQ ID NO:2 or SEQ ID NO: 3.

Another preferred embodiment of the invention refers to a pharmaceutical composition comprising at least one antibody or fragment thereof specific against NK1, NK2 and/or NK3 receptors, or combinations thereof. The antibody used in this invention, preferably, recognises at least a sequence of NK1, NK2 or NK3 receptors, selected among: SEQ ID NO:1, SEQ ID NO:2 or SEQ ID NO: 3. This pharmaceutical composition can also comprise pharmaceutically acceptable carries and/or excipients.

The scope of this invention also includes the use of this pharmaceutical composition for the treatment of cancer. More particularly, this cancer is selected from the group comprising: melanoma, carcinomas, adenocarcinomas, leukaemia, lymphoma, neuroblastomas (or other embryonic or neuroectodermic tumours) and/or sarcomas.

More preferably, the carcinoma is selected from any of the following: breast, including ductal, lung, including small cell and non-small cell, thyroid, including follicular and papillary, digestive system, prostate, cervix, endometrium, ovary, bladder, including endothelial and transitional and/or choriocarcinoma.

In addition, the adenocarcinoma is selected from any of the following: breast, ovary, pancreas, lung and digestive system, including the stomach, and the colon.

On the other hand, leukaemia is preferably human T cell leukaemia, B cell leukaemia, and/or T/NK cell lymphoblastic leukaemia. Lymphomas are preferably of B type, T type, Hodgkin lymphoma and/or Burkitt lymphoma.

The types of sarcoma to be treated by the use of the pharmaceutical composition of the invention are preferably osteosarcomas, rhabomyosarcomas, fibrosarcomas, malignant fibrous histiocytoma, sarcoma of the endometrial stroma and/or Edwing sarcoma.

On the other hand, other neoplasms to be treated by the use of the pharmaceutical composition of the invention are preferably neuroblastoma, as well as other neoplasms of germ cell and/or neuroectodermic origin.

Another preferred embodiment of this invention is the use of the aforementioned pharmaceutical composition for the induction of apoptosis in tumour cells.

An even more preferred embodiment of implementation of this invention is the use of the aforementioned pharmaceutical composition to reduce tumour size, by modification of peritumoural microenvironment, comprising modification of the physiology of the stromal matrix and stromal cells around the tumours, modification of the physiology of the cells of inflammatory and/or immune response, and/or modification of the physiology of the cells of vascularisation in tumour and peritumoural areas. In this regard, the modification of the physiology of the stromal matrix and stromal cells and/or the physiology of the cells involved in the inflammatory and immune response comprises the inhibition of tumour markers. These tumour markers are selected preferably from any of the list: NF-kB, TGF alpha, TGF-beta 1, TGF-beta 2, TGF-beta 3, SPARC, MMP-3, MMP-7, MMP-9, MMP-11, MMP-13, MMP-14 and/or any combinations thereof. In addition, the modification of the physiology of the cells responsible for vascularisation comprises the inhibition of neovascularisation, preferably by the inhibition of vascular endothelial cell proliferation.

The invention also comprises at least one antibody or fragment thereof specific against NK1, NK2 and/or NK3 receptors, or combinations thereof, for use in the treatment of cancer. The antibody used in this invention, preferably, recognises at least a sequence of NK1, NK2 or NK3 receptors, selected among: SEQ ID NO:1, SEQ ID NO:2 or SEQ ID NO: 3.

A preferred form of embodiment of the invention consists of at least one antibody or fragment thereof, specific against NK1, NK2 and/or NK3 cell receptors, or combinations thereof, to induce apoptosis in tumour cells. The antibody used in this invention, preferably, recognises at least a sequence of NK1, NK2 or NK3 receptors, selected among: SEQ ID NO:1, SEQ ID NO:2 or SEQ ID NO: 3.

An additional form of embodiment of this invention comprises at least one antibody or fragment thereof, specific against NK1, NK2 and/or NK3 cell receptors, or combinations thereof, for use in the reduction of tumour size, by modification of peritumoural microenvironment, comprising modification of the physiology of the stromal matrix and stromal cells around the tumours, modification of the physiology of the cells of inflammatory and/or immune response, and/or modification of the physiology of the cells of vascularisation in tumour and peritumoural areas. In this regard, the modification of the physiology of the stromal matrix and stromal cells and/or the physiology of the cells involved in the inflammatory and immune response comprises the inhibition of tumour markers. These tumour markers are selected preferably from any of the list: NF-kB, TGF alpha, TGF-beta 1, TGF-beta 2, TGF-beta 3, SPARC, MMP-3, MMP-7, MMP-9, MMP-11, MMP-13, MMP-14 and/or any combinations thereof. In addition, the modification of the physiology of the cells responsible for vascularisation comprises the inhibition of neovascularisation, preferably by the inhibition of vascular endothelial cell proliferation. The antibody used in this invention preferably recognises at least a sequence of NK1, NK2 or NK3 receptors, selected among: SEQ ID NO:1, SEQ ID NO:2 or SEQ ID NO: 3.

Another embodiment of the invention is a method for the treatment of cancer that comprises administration to a patient of an effective amount of at least an antibody or fragment thereof specific against NK1, NK2 and/or NK3 cell receptors, or combinations thereof, or a pharmaceutical composition as described previously that comprises these antibodies. The antibody used in this invention preferably recognises at least a sequence of NK1, NK2 or NK3 receptors, selected among: SEQ ID NO:1, SEQ ID NO:2 or SEQ ID NO: 3. The types of cancer that can be treated with the method described in this invention are the same as those previously described.

In an even more preferred embodiment of this invention this method is characterised in that it increases the apoptosis of cell tumours by administration to a patient of an effective amount of at least an antibody or fragment thereof specific against NK1, NK2 and/or NK3 cell receptors, or combinations thereof, or a pharmaceutical composition as described previously that comprises these antibodies. The antibody used in this invention preferably recognises at least a sequence of NK1, NK2 or NK3 receptors, selected among: SEQ ID NO:1, SEQ ID NO:2 or SEQ ID NO: 3.

In another additional form of implementation of the invention this method is characterised in that it reduces tumour size by modification of the peritumoural microenvironment and/or the peritumoural inflammatory and/or immune response, as described above.

The preferred method of administration of antibodies or the pharmaceutical compositions containing them in humans is the direct via.

The particular embodiments and the data provided are given by way of illustration and are not intended to be limiting of this invention.

### Example 1. Treatment with NK1, NK2 and NK3 receptor antibodies inhibits proliferation and induces apoptosis in cell lines from different types of cancer.

### Commercial tumour cell lines

Table 1 below details a selection of specific commercial tumour cell lines over which NK1, NK2 and/or NK3 receptor antibodies act. These commercial cell lines were cultured according to the instructions given by the different marketing companies, DSMZ (Deutsche Sammlung von Mikrorganismen und Zellkulturen), ICLC (Interlab Cell line collection -CBA- Genoa), and ECACC (European Collection of Cell Cultures). Briefly, the commercial tumour cell lines were maintained in culture in DMEM medium (Dulbecco's modified Eagle's medium, simple modification of the Eagle's basal medium) or RPMI 1640 (Roswell Park Memorial Institute) (Gibco, Barcelona, Spain) supplemented with 10% of foetal bovine serum (FBS) inactivated by treatment with heat. In addition, 1% of antibiotics-antimycotics (Invitrogen) were added to the medium. The cell lines were sown in culture bottles with a surface of 75-cm² (Falcon, Heidelberg, Germany). The culture medium was renewed every two days. The cells were incubated in culture chambers at a temperature of 37°C and in a wet atmosphere with 95% of air and 5% of CO₂. After 6 days from the cell sowing, these were collected by treatment with trypsin (0.05 and 0.02% EDTA with no calcium or magnesium; Sigma-Aldrich, Madrid, Spain). All cells were deprived from serum the night before performing the tests unless otherwise indicated. In all these cells lines, the presence of NK1, NK2 and NK3 receptors was verified, evidencing the antitumoural action of anti-NK1, anti-NK2 and anti-NK3 antibodies.

**Table 1. Tumour cell lines used to demonstrate the antitumoural action of NK1, NK2 and NK3 receptor antibodies.**

| **Type of tumour** | **Cell line** | **Marketing company** |
|---|---|---|
| Human colon adenocarcinoma | SW-403 | DSMZ |
| Human breast adenocarcinoma | MCF-7 | DSMZ |
| Human ovarian adenocarcinoma | EFO-27 | DSMZ |
| Human pancreatic adenocarcinoma | CAPAN-1 | DSMZ |
| Human pancreatic adenocarcinoma | PA-TU 8902 | DSMZ |
| Human lung adenocarcinoma | DV-90 | DSMZ |
| Human gastric adenocarcinoma | 23132/87 | DSMZ |
| Human cervix carcinoma | KB | DSMZ |
| Human cervix carcinoma | BT-B | DSMZ |
| Human endometrium carcinoma | AN3-CA | DSMZ |
| Human breast carcinoma | MT-3 | DSMZ |
| Human breast carcinoma | MDA-MB-468 | DSMZ |
| Human ovarian carcinoma | COLO-704 | DSMZ |
| Human ovarian carcinoma | FU-OV-1 | DSMZ |
| Human prostate carcinoma | 22RV1 | DSMZ |
| Human non-small cell lung carcinoma | HCC-44 | DSMZ |
| Human non-small cell lung carcinoma | COR-L23 | ECACC |
| Human small cell lung carcinoma | H-209 | DSMZ |
| Human small cell lung carcinoma | H-1963 | DSMZ |
| Human small cell lung carcinoma | H69 | ECACC |
| Human lung carcinoma | A-427 | DSMZ |
| Human thyroid carcinoma | 8505C | DSMZ |
| Human metastasizing papillary thyroid carcinoma | B-CPAP | DSMZ |
| Human bladder carcinoma | 5637 | DSMZ |
| Ductal breast carcinoma | BT-474 | DSMZ |
| Human follicular thyroid carcinoma | TT2609-C02 | DSMZ |
| Human transitional bladder carcinoma | RT-4 | DSMZ |
| Human urothelial bladder carcinoma | 647-V | DSMZ |
| Human choriocarcinoma | AC-1M32 | DSMZ |
| Human fibrosarcoma | HT-1080 | DSMZ |
| Human glioma | GAMG | DSMZ |
| Human malignant fibrous histiocytoma | U-2197 | DSMZ |
| Human T-/NK cell leukaemia | YT | DSMZ |
| B lymphoblastic leukaemia (peripheral human B lymphoblastoid cells) | SD1 | DSMZ |
| Human T lymphoblastic leukaemia | BE-13 | DSMZ |
| Human B lymphoma | BC-1 | DSMZ |
| Human B lymphoma | SU-DHL-16 | DSMZ |
| Human Burkitt lymphoma | CA-46 | DSMZ |
| Human Burkitt lymphoma | KM-H2 | DSMZ |
| Human T lymphoma | DERL-7 | DSMZ |
| Human T lymphoma | SUP-T1 | DSMZ |
| Human melanoma | MEL HO | DSMZ |
| Human melanoma | COLO679 | DSMZ |
| Human melanoma, metastasis in lymph node | COLO858 | ICLC |
| Human multiple myeloma | COLO-677 | DSMZ |
| Human neuroblastoma | KELLY | DSMZ |
| Human neuroblastoma | IMR-32 | DSMZ |
| Human neuroblastoma, bone marrow | SKN-BE 2 | ICLC |
| Human osteosarcoma | MG-63 | ICLC |
| Human osteosarcoma | CAL-72 | DSMZ |
| Human rhabdomyosarcoma | A-204 | DSMZ |
| Human retinoblastoma | Y-79 | DSMZ |
| Human retinoblastoma | WERI-Rb-1 | DSMZ |
| Human Edwing sarcoma | MHH-ES-1 | DSMZ |
| Human endometrial stroma sarcoma | ESS-1 | DSMZ |

| | | |
|---|---|---|
| DSMZ: Deutsche Sammlung von Mikrorganismen und Zellkulturen. ICLC: Interlab Cell line collection -CBA- Genoa. ECACC: European Collection of Cell Cultures. | | |

### Western Blot

For Western Blot assays, samples of each commercial cell line were tested as shown in Table 1. This technique was used to establish that all cell lines, both commercial and obtained directly from human samples (primary cultures), evidenced the presence of NK1, NK2 and NK3 receptors. In brief, the extraction of total proteins was performed from the samples obtained from tumour culture cells. The cells were lysed by methods commonly known in the state of the art and their concentration was measured using a commercial kit "Protein Assay" from Bio-Rad (Bio-Rad Laboratories, S.A. Madrid), following the manufacturer's instructions for it. For each sample, 50 mg of protein were separated by 10% gel electrophoresis SDS-polyacrylamide and transferred electrophoretically to polyvinylidene fluoride (PVDF) membranes. These membranes were incubated overnight in a blocking solution (5% of skimmed milk in phosphate buffer (PBS) with 0.1% of Tween-PBST), followed by incubation overnight with primary antibodies diluted 1/4000 in PBST buffer:
anti-NK1: antibody recognising the domain corresponding to the carboxyl-terminal region of the NK1 receptor in amino acids 393-407 (SEQ ID NO: 1), obtained from the company Sigma-Aldrich, under product number S8305.
anti-NK2: recognises the sequence SEQ ID NO: 2 of the NK2 receptor obtained from Sigma-Aldrich, under product number HPA012084.
anti-NK3: recognises the sequence SEQ ID NO: 3 of the NK3 receptor obtained from Sigma-Aldrich, under product number HPA009418.

The membranes were then washed with phosphate buffer saline (PBS) and in the presence of detergent Tween-20 (PBST) and incubated with a secondary antibody conjugated with radish peroxidase for 2 hours at room temperature (dilution 1:10000). To confirm that the same amount of protein had been loaded, the membranes were incubated with anti-β-tubulin monoclonal antibody. The detection of antibodies was performed with a chemiluminiscence reaction (ECL Western Blot detection; Amersham Life Science, United Kingdom).

### Preparation of cell blocks included in paraffin and performing immunohistochemistry of NK1, NK2 and NK3 receptors.

The immunohistochemistry technique allows for molecule detection by application of a primary antibody specific for an epitope of these molecules that is evidenced by the application of a secondary antibody that recognises an epitope of the primary antibody and includes a viewing system that can be fluorescent or chromogenic (a chromogenic view method was used in this invention). Therefore, the immunohistochemistry method allows for viewing these molecules and assaying their exact location in the cells or in the extracellular stroma, as the study can be performed over histological sections. The immunohistochemistry techniques were used to establish that all cell lines, both primary and commercial, evidenced the presence of NK1, NK2 and NK3 receptors.

A sample of each of the cultures of the cell lines was centrifuged (5 minutes at 1,500 rpm) and the pellet obtained was dehydrated by treatment at growing concentrations of ethanol and finally in xylol. Then these dehydrated samples were included in paraffin creating a block of cells. These paraffin blocks were cut in a microtome at a thickness of 5 micra and deparaffinised by sinking in xylol, to be subsequently hydrated through a number of solutions containing decreasing concentrations of ethanol and be finally sank in water. Then, these samples were subject to treatment in a pressure cooker in citrate buffer 10x at pH 6.0, to obtain a higher exposure of antigens. Subsequently, the samples were cooled at room temperature for 10 minutes. The endogenous peroxidase activity was blocked with 3% hydrogen peroxide for 30 minutes at room temperature. After washing the samples with 0.05 M of Tris buffer, they were incubated with swine non-immune serum at 10% for 30 minutes at room temperature.

Subsequently, to verify the expression of NK1, NK2 and NK3 receptors, the cell samples were incubated in the presence of anti-NK1, anti-NK2 or anti-NK3 antibodies (Sigma-Aldrich) diluted 1:500 overnight at 4°C. After this time, they were washed in 0.05 M Tris buffer at room temperature. The next step was the addition of reagents Envision System-HRP (Dako) for 30 minutes at room temperature. After this time, the samples were washed again in 0.05 M Tris buffer, and immunoreactivity was viewed by light microscopy with a chromogenic solution with 3,3'-diaminobenzidine (DAB+; Dako, USA). To distinguish the cell nuclei, these were stained slightly with hematoxylin. The negative controls used were samples not incubated with the primary anti-NK1, anti-NK2 or anti-NK3 antibody, respectively, that was replaced by non-immune serum. All samples were evaluated. In addition, a negative control was included, where the primary antibody was omitted, and was replaced by non-immune serum.

### Proliferation assays

Cell proliferation was evaluated using the compound tetrazolium 3-(4, 5-dimethylthiazol-2-yl)-5-(3-carboxymethoxyphenyl) 2-(4-sulphophenyl)-2H-tetrazolium (MTS) according to the instructions given by the manufacturer (Kit "CellTiter 96 Aqueous One-Solution Cell Proliferation Assay" Promega, USA; full protocol of use available at url: http://www.promega.com/resources/protocols/technical-bulletins/0/celltiter-96-aqueous-one-solution-cell-proliferation-assay-system-protocol/). The number of cells was measured using a Coulter meter. The tumour cell culture plaques included a blank sample (free from cells) and a control sample (containing 10⁴ cell/mL). The culture plaques were cultured in the presence of growing concentrations of anti-NK1, anti-NK2 or anti-NK3 monoclonal antibodies. To obtain staining in proliferation assays, 20 µL of MTS were added to each of the wells of the cell culture plaques 90 minutes before performing reading of the samples in a multiscanner spectrophotometer (TECAN Spectra classic, Barcelona, Spain) at 492 nm (wavelength of the assay) and 690 nm (reference wavelength). The different doses were tested in duplicate and each test was performed in triplicate.

### Determination of apoptosis: staining with DAPI (4',6-diamidine-2-phenylindol).

The apoptotic morphology was assayed by fluorescence microscopy after staining with DAPI. For determination if the NK1, NK2 and NK3 receptor antibodies are able to induce apoptosis in tumour cell cultures, staining was performed with DAPI. In brief, after treatment with NK1, NK2 and NK3 receptor antibodies, the cells were fixed in 4% paraformaldehyde. After a second washing in PBS, the cells were incubated in DAPI solution (Sigma-Aldrich) at a concentration of 1/1000 for 30 minutes in darkness. Subsequently, the cells were inspected under a fluorescence microscope (Zeiss, Oberkochen, Germany). Apoptotic cells were identified by typical characteristics of apoptosis (e.g., nuclear condensation, nuclear vacuolisation, loss of integrity of the cell membrane and formation of apoptotic antibodies). The number of apoptotic cells was counted, repeating the count in three different slides.

The NK1, NK2 and NK3 receptor antibodies used in this invention are mouse polyclonal antibodies obtained from Sigma-Aldrich mentioned above.

For the purpose of analysing whether cell death or apoptosis occurred as a result of modification of the activity of NK1, NK2 and/or NK3 receptors by the treatment with anti-NK1, anti-NK2 or anti-NK3 antibodies, first the cell cultures from commercial cell lines related to human cancers were sown according to recommendations from the manufacturers and the common laboratory use (see section "Commercial Tumour Cell Lines"). When the cell cultures evidenced confluence -the cells stop dividing, the culture becomes saturated, inhibition occurs by contact in cells in monolayer and consumption of the medium in cells in suspension- of about 85%, the serum was removed and they were cultured overnight in serum-free culture medium. Then these cultures were treated with anti-NK1, anti-NK2 or anti-NK3 antibodies at different concentrations.

To establish the presence of apoptosis, the apoptotic morphology was analysed, identifying apoptotic cells by the typical characteristics of apoptosis, such as nuclear condensation, nuclear vacuolisation, loss of integrity of the nuclear membrane and formation of apoptotic bodies. All these characteristics were analysed by fluorescence microscopy after staining with DAPI (Roche).

To confirm the presence of apoptosis, supernatants of the cell cultures of tumour lines treated with anti-NK1, anti-NK2 or anti-NK3 antibodies were analysed, that included cells detached from the culture plaques. To check whether these cells had detached from the Petri dish due to apoptosis thereof, DAPI staining was performed of the precipitates of the supernatants obtained from the culture media. These precipitates included the presence of cell detritus together with cells with clear apoptotic morphology.

The analysis of nuclear morphology and apoptosis was performed every 24 hours for 96 hours. The different doses were tested six times and each test was performed in triplicate. In each case a control sowing was performed in parallel, that was subject to the same conditions as those treated with anti-NK1, anti-NK2 or anti-NK3 antibody, except for exposure to this treatment.

All commercial cell lines of human cancer analysed (see full list in Table 1) evidenced the expression for NK1, NK2 and NK3 receptors, verified by both the Western-Blot technique and by immunohistochemistry.

All commercial cell lines of human cancer analysed (see full list in Table 1) were subject to treatment with different concentrations of anti-NK1, anti-NK2 or anti-NK3 antibodies, and the presence of cells with typical characteristics of apoptosis was evidenced in all by DAPI staining. A dose-dependent increase was seen in the apoptosis of these commercial tumour cell lines, since growing amounts of anti-NK1, anti-NK2 or anti-NK3 antibody increased the number of cells with characteristics typical of apoptosis. The number of cells with characteristics typical of apoptosis increased over the observation period (on a time-dependent basis). These results were not seen in the control cultures (that were subject to a procedure under conditions identical to the previous, except for exposure to treatment with anti-NK1, anti-NK2 o anti-NK3 antibody).

In all commercial cell lines of the human tumours analysed (see full list in Table 1) and subject to treatment with different concentrations of anti-NK1, anti-NK2 or anti-NK3 antibodies for different times, 48, 72 and 96 hours, a time and dose-dependent inhibition of proliferation was seen: a higher inhibition was evidenced as the dose of anti-NK1, anti-NK2 or anti-NK3 antibodies and the time of exposure were increased. Figures 1 to 7 show examples of inhibition of proliferation of cell cultures from different lines of adenocarcinoma, such as, for instance, colon adenocarcinoma (Fig 1), breast adenocarcinoma (Fig 2), or ovarian adenocarcinoma (Fig 3), pancreatic adenocarcinoma (Fig 4 and 5), lung adenocarcinoma (Fig 6) and gastric adenocarcinoma (Fig 7).

Analyses were also performed on the inhibition of proliferation of cultures of cell lines from different carcinomas by treatment thereof with anti-NK1, anti-NK2 or anti-NK3 antibodies at different concentrations and for the times mentioned above, 48, 72 and 96 hours. The results obtained are shown in Figures 8 to 28. The commercial tumour cell lines of carcinomas used for obtaining these results have been: of the cervix carcinoma (Fig. 8 and 9), endometrium carcinoma (Fig. 10), breast carcinoma (Fig. 11 and 12), ovary carcinoma (Fig. 13 and 14), prostate carcinoma (Fig. 15), non-small cell lung carcinoma (Fig. 16 and 17), small-cell lung carcinoma (Fig. 18-20), lung carcinoma (Fig. 21), thyroid carcinoma (Fig. 22), metastasizing human papillary thyroid carcinoma (Fig. 23), bladder carcinoma (Fig. 24), ductal carcinoma (Fig. 25), human follicular thyroid carcinoma (Fig. 26), transitional of the bladder carcinoma (Fig. 27) and urothelial of the bladder carcinoma (Fig. 28). As shown in these figures, all carcinoma cell lines show an inhibition of proliferation in the presence of anti-NK1, anti-NK2 or anti-NK3 antibodies.

Cell lines of choriocarcinoma (Fig 29), fibrosarcoma (Fig 30), glioma (Fig 31) and malignant fibrous histiocytoma (Fig 32) also evidenced inhibition of proliferation by treatment with anti-NK1, anti-NK2 or anti-NK3 antibodies, evidencing that the treatment of tumour lines with these antibodies leads to a reduction of the tumour.

Cell lines from different types of leukaemia, such as for instance, human T/NK cell leukaemia (Fig 33), B lymphoblastic leukaemia (Fig 34) and T lymphoblastic leukaemia (Fig 35), as well as cells lines of lymphomas, human B lymphoma (Fig 36 and 37), Burkitt lymphoma (Fig. 38), Hodgkin lymphoma (Fig 39), T lymphoma (Fig 40 and 41), cell lines from melanoma and multiple myeloma (Figs 42 and 45), also evidenced proliferation inhibition, by the treatment with anti-NK1, anti-NK2 or anti-NK3 antibodies, evidencing again that this treatment leads to a tumour reduction.

Additional tests performed in culture cells from neuroblastomas (Figs 46 to 48), from osteosarcomas (Fig 49 and 50), from rhabdomyosarcoma (Fig 51), from retinoblastoma (Fig 52 and 53), from Edwing sarcoma (Fig 54) and from endometrial stromal sarcoma (Fig 55), also evidenced inhibition of proliferation, by the treatment with anti-NK1, anti-NK2 or anti-NK3 antibodies, inducing a tumour size reduction.

In addition, as mentioned above, by staining with DAPI, in all aforementioned cell lines was verified that the increase in apoptosis of these commercial tumour cell lines was time and dose-dependent, as growing amounts of anti-NK1, anti-NK2 or anti-NK3 antibodies, as well as growing periods of exposure, increased the number of cells with characteristics typical of apoptosis.

### Example 2. Modification of the peritumoural microenvironment by the treatment with anti-NK1, NK2 and/or NK3 antibodies.

As discussed in this invention, not only the molecular mechanisms of tumour cells are involved in the genesis and development of cancer, but also stromal cells, inflammation and interactions between tumour and stromal cells and inflammation are very important (McAllister SS, et al. 2010; Ikushima H, Miyazono K, 2010). Example 1 shows that the treatment with NK1, NK2 and NK3 receptor antibodies induces apoptosis in several commercial tumour cell lines. This example shows that, in addition, the treatment with antibodies induces changes and modifications in the physiology of the cells that form the tumour microenvironment, which are beneficial for the treatment of cancer, for instance in fibroblasts, in inflammation cells (mononuclear and polymorphonuclear), and in the vascular endothelium. Factors with well-documented importance in the scientific literature and with expression modified in the presence of agonists (for instance, substance P) of NK1, NK2 or NK3 receptors and in the presence of antibodies against these receptors are involved in this physiology.

To verify that NK1, NK2 or NK3 receptor antagonists, for instance, substance P, induce physiological and functional changes in stromal cells -fibroblasts- and in inflammation cells similar to those seen in these same cells in peritumoural areas and that these effects are reverted by NK1, NK2 or NK3 receptor antibodies, the presence of different markers (molecules) in the cells forming the tumoural microenvironment has been analysed (see list below in Table 2 and in Table 3) in primary human fibroblast (PHF) culture cells, human primary mononuclear and polymorphonuclear blood cells, in the presence of substance P, NK1, NK2 or NK3 receptor agonist, as well as in the presence of this agonist and of anti-NK1, anti-NK2 or anti-NK3 antibodies. It was also verified that the antibodies against these receptors inhibit proliferation of microvascular endothelial cells of the commercial line of juvenile foreskin (PromoCell GmH, Sickingenstraße 63/65, D-69126 Heidelberg, Germany; reference C-12210).

### Isolation and culture of primary human fibroblasts (PHF)

PHF were obtained and purified from samples obtained from the skin dermis as described in De Bari et al. (De Bari et al., 2001). In brief, small pieces of skin dermis were digested in a hyaluronidase solution (Sigma-Aldrich, St. Louis, MO, US) at a concentration of 1 mg/mL for 15 minutes at 37°C and subsequently treated with 6 mg/mL of type IV collagenase (Invitrogen) for 2 hours at 37°C. Then the cells were washed, resuspended in DMEM culture medium with a high concentration of glucose (Dulbecco's Modified Eagle's Medium, Invitrogen) supplemented with 1% of antibiotics-antimicotics (Invitrogen) and with 1% of sodium pyruvate (Invitrogen), and sown in 6-well culture plaques at a concentration of 10,000 cells per square centimetre. When the cells reached confluence, the adherent cells were separated using 0.5% sterile trypsin (Invitrogen) and were used between runs 3 and 9. For stimulation of substance P and exposure to anti-NK1, anti-NK2 and anti-NK3 antibodies, PHF were sown in 24-well plaques at a concentration of 25,000 cells per well.

### Isolation and culture of human mononuclear and polymorphonuclear blood cells

Heparinised blood from healthy donors was centrifuged, separating the different components thereof. Red blood cells remain in the bottom of the tube. Above them, there is a small whitish layer formed by white blood cells and above, in the upper part of the tube, blood plasma. The white blood cell layer was distributed in a culture plate of 24 wells, adding to each of them the same concentration of blood plasma supplemented with 1% of antibiotics-antimicotics (Invitrogen). About 1000 cells (PHF) from the skin dermis, obtained as described above, were added to each well.

### Culture and proliferation assays performed on endothelial cells in the presence of NK1, NK2 and NK3 receptors antibodies

Cell cultures from a commercial line formed by microvascular endothelial cells from juvenile foreskin were also used (PromoCell GmbH, Sickingenstraße 63/65, D-69126 Heidelberg, Germany; reference C-12210). Proliferation assays were performed similarly to example 1 (see section "proliferation assays"), in the presence of NK1, NK2 and NK3 receptors antibodies.

As discussed above, to analyse the modification of the peritumoural microenvironment by treatment with anti-NK1, NK2 and/or NK3 antibodies, the presence of specific cell markers has been analysed in PHF. These markers, such as for instance TGF-alpha, SPARC and metalloproteases (MMPs) have been associated with tumour progression (Coussens L and Werb Z. 2002; Carmeliet P and RK Jain. 2000). Specifically, with regard to MMPs, there are several drugs known as inhibitors thereof that are currently tested for the treatment of cancer.

For the purpose of analysing the expression of these cell markers in PHF cultures in the presence of the NK1, NK2 and NK3 receptor agonist and in the presence of anti-NK1, NK2 or NK3 antibodies, these cells were cultured in the presence of substance P (NK1, NK2 and NK3 receptor agonist) at a concentration of 1 µM (product no. S6883; Sigma-Aldrich). In other wells and simultaneously these cells were cultured with substance P at the same concentration and simultaneously together with anti-NK1, anti-NK2 or anti-NK3 antibodies (Sigma-Aldrich) at a 1/100 concentration. After 48 hours of culture, the cells were recollected and cell blocks were built for immunohistochemistry assays, as explained in example 1. In the immunohistochemistry assays, the expression of the following markers was assayed: TGF-alpha (SAB4502953), TGF-beta 1 (SAB4502954), TGF-beta 2 (SAB4502956), TGF-beta 3 (SAB4502957), SPARC (HPA002989) MMP-3 (HPA007875); MMP-7 (SAB4501894), MMP-9 (SAB4501896), MMP-11 (SAB4501898), MMP-13 (SAB4501900) and MMP-14 (SAB4501901), by specific antibodies against them. For performing these immunohistochemistry assays, all antibodies were mouse polyclonal antibodies obtained from Sigma Aldrich, used at a 1/1000 concentration. The immunohistochemistry techniques were performed as described in Example 1. The results are summarised in Table 2, that gives the type of marker and the mean percentage of cells with immunostaining for this marker.

To assess immunostaining, six serial sections were made of each block placed over slides for performing the immunohistochemistry technique for each marker. Therefore, the immunohistochemical assay was performed six times for each marker in each case. In each of the six sections, cell count was performed in 20 fields of high magnification (400x) using a microscope of Olympus brand (model CX31). In each field, the total cell number and the number of cells with immunostaining were counted, subsequently measuring the percentage of cells with this immunostaining. Table 2 and 3 show the mean percentage of cells with immunostaining for each marker in each block performed.

The results evidenced the presence of all markers tested in the samples from PHF cell cultures treated only with substance P (NK1, NK2 and NK3 receptor agonist). This evidences that the NK1, NK2 and NK3 receptor agonist induces in primary human fibroblasts immunophenotype changes similar to those found in peritumoural areas. On the contrary, no immunostaining, sign of absence of expression, was seen for the cell cultures treated jointly with substance P and with each anti-NK1, NK2 or NK3 antibody. This demonstrates that exposure to the NK receptor agonist substance P can induce in PHF expression of markers characteristic of these cells in peritumoural areas and that the use of NK1, NK2 and NK3 receptor antibodies can revert the expression of these markers that, as explained, are characteristic of peritumoural areas, characteristic of peritumoural microenvironment and are involved in the development, enlargement and progression of tumours.

**TABLE 2. Percentage (*) of fibroblast cells with expression for immunohistochemical markers important in the tumour microenvironment after exposure to substance P and anti-NK1, NK2 and NK3 antibody.**

| | **Percentage of cells with expression for each marker** | | | |
|---|---|---|---|---|
| **Marker** | **Substance P (1µM)** | **anti-NK1 (1/100) and Substance P (1µM)** | **anti-NK2 (1/100) and Substance P (1µM)** | **anti-NK3 (1/100) and Substance P (1µM)** |
| TGF-alpha | 16.5% | 0% | 0% | 0% |
| TGF-beta 1 | 18.4% | 0% | 0% | 0% |
| TGF-beta 2 | 24.6% | 0% | 0% | 0% |
| TGF-beta 3 | 23.1% | 0% | 0% | 0% |
| SPARC | 16% | 0% | 0% | 0% |
| MMP-3 | 33.9% | 0% | 0% | 0% |
| MMP-7 | 46.2% | 0% | 0% | 0% |
| MMP-9 | 35.5% | 0% | 0% | 0% |
| MMP-11 | 7.8% | 0% | 0% | 0% |
| MMP-13 | 17.1% | 0% | 0% | 0% |
| MMP-14 | 47.3% | 0% | 0% | 0% |

| | | | | |
|---|---|---|---|---|
| (*)The percentage is the mean of the percentages found (in each case the immunohistochemistry technique was performed six times and 20 high-magnification fields were tested). | | | | |

The modification of the peritumoural microenvironment by treatment with anti-NK1, NK2 and/or NK3 antibodies has also been analysed determining the involvement of mononuclear and polymorphonuclear blood cells in this tumoural microenvironment. There are evidences of the influence of inflammation in the genesis and development of cancer (De Visser KE, et al. 2006; McAllister SS, et al. 2010; Ikushima H, et al. 2010). Several mediators enhancing interaction between stroma, inflammatory cells and tumour cells are also known, that contribute to the development of the latter (Li W, et al. 2007). These mediators include most remarkably TGF-beta and NF-kB. For the purpose of analysing the presence of cell markers specific for these cells and their modification in the presence of anti-NK1, NK2 and/or NK3 antibodies, in this invention mononuclear and polymorphonuclear cells were incubated in the presence of PHF obtained by the aforementioned methods and with the presence of Substance P at a concentration of 1 µM or in the presence of Substance P (1 µM) together with anti-NK1, anti-NK2 or anti-NK3 antibodies at a concentration of 1/100.

After incubation for 48 hours, the cells were collected and for the immunohistochemistry assays they were included in paraffin blocks as described in Example 1. Histology sections were subsequently made of each block, assaying expression by immunohistochemistry of cell markers TGF-beta (SAB4502956) and NF-kB (SAB4501992) by specific antibodies against them. All antibodies used were mouse polyclonal antibodies obtained from Sigma Aldrich and used at a concentration of 1/1000. The immunohistochemistry techniques were performed as described in Example 1.

The results are summarised in Table 3 and demonstrated the presence of TGF-beta and NF-kB in mononuclear and polymorphonuclear cells of samples from cultures treated only with Substance P (NK1, NK2 and NK3 receptor agonist). On the contrary, no immunostaining was seen for the different markers in samples of mononuclear and polymorphonuclear cell cultures treated with Substance P and with NK1, NK2 or NK3 receptor antibodies. This shows that the exposure of mono and polymorphonuclear inflammation blood cells to NK1, NK2 and NK3 receptors (Substance P) induces expression of TGF-beta and NF-kB that are mediators of proven importance in the genesis of the microenvironment necessary for the development, enlargement and progression of tumours. On the contrary, with the use of NK1, NK2 and NK3 antibody receptors, the expression of these markers can be reverted, which shows the ability of these antibodies to inhibit the genesis of these mediators and, therefore, prevent the development of a tumour microenvironment that allows for the enlargement and progression of tumours.

**TABLE 3. Percentage (*) of inflammation blood cells (mono and polymorphonuclear) with expression for immunohistochemical markers important in the tumour microenvironment after exposure to Substance P and anti-NK1, NK2 and NK3 antibody.**

| | **Percentage of mononuclear cells with expression for each marker** | | | |
|---|---|---|---|---|
| **Marker** | **Substance P (1µM)** | **anti-NK1 (1/100) and Substance P (1µM)** | **anti-NK2 (1/100) and Substance P (1µM)** | **anti-NK3 (1/100) and Substance P (1µM)** |
| TGF-beta | 16.4% | 0% | 0% | 0% |
| NF-kB | 25.7% | 0% | 0% | 0% |

| | **Percentage of polymorphonuclear cells with expression for each marker** | | | |
|---|---|---|---|---|
| **Marker** | **Substance P (1µM)** | **anti-NK1 (1/100)** | **anti-NK2 (1/100)** | **anti-NK3 (1/100)** |
| TGF-beta | 36.8% | 0% | 0% | 0% |
| NF-kB | 17.5% | 0% | 0% | 0% |

| | | | | |
|---|---|---|---|---|
| (*)The percentage is the mean of the percentages found (in each case the immunohistochemistry technique was performed five times). | | | | |

Finally, it is known that angiogenesis is essential for the development and maintenance of tumours (Hanahan D, Weinberg RA. Cell. 2000). In this regard, the use of monoclonal antibodies against the extracellular domain of VEGFR is clinically approved for the treatment of several types of cancers. In this regard, the modification of the peritumoural microenvironment by inhibition of vascular endothelial cell growth with treatment with anti-NK1, anti-NK2 and/or anti-NK3 antibodies has been also analysed. For this, following the method explained in example 1 (section of proliferation tests) the commercial line of microvascular endothelial cells called C-12210 was cultures in the presence of increasing concentrations of anti-NK1, NK2 or NK3 antibodies, and a time and dose-dependent inhibition was seen in their proliferation (Figure 56). Therefore, anti-NK1, anti-NK2 or anti-NK3 antibodies inhibit proliferation of endothelial cells. Proliferation of endothelial cells is a key element in the development of the neovascularisation necessary for tumours to receive a blood supply (and therefore of oxygen, nutrients, amongst other necessary elements) sufficient for maintaining growth and progression.

### REFERENCES

● Adams GP, Weiner LM. Monoclonal antibody therapy of cancer. Nat Biotechnol. 2005 Sep;23(9):1147-57.
● Barker R. Tachykinins, neurotrophism and neurodegenerative diseases: a critical review on the possible role of tachykinins in the aetiology of CNS diseases. Neurosci. Res., 1996, 7, 187-214.
● Berzofsky JA, et al. Progress on new vaccine strategies for the immunotherapy and prevention of cancer. J Clin Invest. 2004; 113:1515-1525.
● Berzofsky JA, Terabe M, Oh S, Belyakov IM, Ahlers JD, Janik JE, Morris JC.
● Bigioni M, Benzo A, Irrissuto C, Maggi CA, Goso C. Role of NK-1 and NK-2 tachykinin receptor antagonism on the growth of human breast carcinoma cell line MDA-MB-231. Anticancer Drugs. 2005,16(10):1083-9.
● Bunn PA Jr, Chan D, Stewart J, Gera L, Tolley R, Jewett P, Tagawa M, Alford C, Mochzuki T, Yanaihara N. Effects of neuropeptide analogues on calcium flux and proliferation in lung cancer cell lines. Cancer Res. 1994;54(13):3602-10.
● Carmeliet P, Jain RK. Angiogenesis in cancer and other diseases. Nature. 2000 Sep 14;407(6801):249-57.
● Cox G, Jones JL, O'Byrne KJ. Matrix metalloproteinase 9 and the epidermal growth factor signal pathway in operable non-small cell lung cancer. Clin Cancer Res. 2000;6(6):2349-55.
● Coussens LM, Werb Z. Inflammation and cancer. Nature. 2002; 19-26;420(6917):860-7.
● De Bari C, Dell'Accio F, Tylzanowski P, Luyten FP. Multipotent mesenchymal stem cells from adult human synovial membrane. Arthritis Rheum. 2001;44(8):1928-42.
● de Visser KE, Eichten A, Coussens LM. Paradoxical roles of the immune system during cancer development. Nat Rev Cancer. 2006;6(1):24-37.
● Doi T, Kamo I, Imai S, Okanishi S, Ishimaru T, Ikeura Y, Natsugari H. Effects of TAK-637, a tachykinin receptor antagonist, on lower urinary tract function in the guinea pig. Eur J Pharmacol. 1999;383(3):297-303.
● Giardina GA, Gagliardi S, Martinelli M. Antagonists at the neurokinin receptors- Recent patent literature. IDrugs. 2003; 6(8):758-72.
● Hanahan D, Weinberg RA. The hallmarks of cancer. Cell. 2000 Jan 7;100(1):57-70.
● Ikushima H, Miyazono K. TGFbeta signalling: a complex web in cancer progression. Nat Rev Cancer. 2010;10(6):415-24.
● Kramer MS, Cutler N, Feighner J, Shrivastava R, Carman J, Sramek JJ, Reines SA, Liu G, Snavely D, Wyatt-Knowles E, Hale JJ, Mills SG, MacCoss M, Swain CJ, Harrison T, Hill RG, Hefti F, Scolnick EM, Cascieri MA, Chicchi GG, Sadowski S, Williams AR, Hewson L, Smith D, Carlson EJ, Hargreaves RJ, Rupniak NM. Distinct mechanism for antidepressant activity by blockade of central substance P receptors. Science. 1998. 11;281(5383):1640-5.
● Lin WW, Karin M. A cytokine-mediated link between innate immunity, inflammation, and cancer. J Clin Invest. 2007 May;117(5):1175-83.
● Maggi CA, Patacchini R, Rovero R, Giachetti A. Tachykinin Receptor and Tachykinin Receptor Antagonist. Journal of Autonomic Pharmacology. 1993 (13):23-93.
● McAllister SS, Weinberg RA. Tumor-host interactions: a far-reaching relationship. J Clin Oncol. 2010;28(26):4022-8.
● Muñoz M, Rosso M, Robles-Frias MJ, Salinas-Martin MV, Rosso R, González-Ortega A, Coveñas R. The NK-1 receptor is expressed in human melanoma and is involved in the antitumor action of the NK-1 receptor antagonist aprepitant on melanoma cell lines. Lab Invest. 2010; 90(8):1259-69
● Orosz A, Schrett J, Nagy J, Bartha L, Schön I, Nyéki O. New short-chain analogs of a substance-P antagonist inhibit proliferation of human small-cell lung-cancer cells in vitro and in vivo. Int J Cancer. 1995;60(1):82-7.
● Palma C, Maggi CA. The role of tachykinins via NK1 receptors in progression of human gliomas. Life Sci. 2000;67(9):985-1001.
● Quartara L, Maggi CA. The tachykinin NK1 receptor. Part II: Distribution and pathophysiological roles. Neuropeptides. 1998;32(1):1-49.
● Rosso M, Robles-Frias MJ, Coveñas R, Salinas-Martin MV, Muñoz M. The NK-1 receptor is expressed in human primary gastric and colon adenocarcinomas and is involved in the antitumor action of L-733,060 and the mitogenic action of substance P on human gastrointestinal cancer cell lines. Tumour Biol. 2008;29(4):245-54.
● Singh D, Joshi DD, Hameed M, Qian J, Gascón P, Maloof PB, Mosenthal A, Rameshwar P. Increased expression of preprotachykinin-I and neurokinin receptors in human breast cancer cells: implications for bone marrow metastasis. Proc Natl Acad Sci U S A. 2000;97(1):388-93.
● Welt S, Divgi CR, Scott AM, Garin-Chesa P, Finn RD, Graham M, Carswell EA, Cohen A, Larson SM, Old LJ, et al. Antibody targeting in metastatic colon cancer: a phase I study of monoclonal antibody F19 against a cell-surface protein of reactive tumor stromal fibroblasts. J Clin Oncol. 1994 Jun;12(6):1193-203.

## Claims

1. Use of at least one antibody or fragment thereof, specific against NK1, NK2 and/or NK3 cell receptors, or combinations thereof, for the manufacture of a medicament or a pharmaceutical composition for the treatment of cancer.

2. Use of at least one antibody or fragment thereof, specific against NK1, NK2 and/or NK3 cell receptors, or combinations thereof, for the manufacture of a medicament or a pharmaceutical composition for the induction of apoptosis in tumour cells.

3. Use of at least one antibody or fragment thereof, specific against NK1, NK2 and/or NK3 cell receptors, or combinations thereof, for the manufacture of a medicament or a pharmaceutical composition to reduce tumour size.

4. Use according to claim 3, **characterised in that** the reduction of tumour size is performed by modifying the peritumoural microenvironment.

5. Use according to claim 4 **characterised in that** the modification of the peritumoral microenvironment comprising the modification of the physiology of the stromal matrix and the stromal cells and/or modification of the physiology of cells involved in inflammatory and/or immune response and/or modification of the physiology of the cells responsible for vascularisation.

6. Use according to claim 5 **characterised in that** the modification of the physiology of the stromal matrix and stromal cells and/or the physiology of the cells involved in the inflammatory and immune response comprises the inhibition of tumour markers.

7. Use according to claim 6 where tumour markers are selected preferably from any of the list: NF-kB, TGF alpha, TGF-beta 1, TGF-beta 2, TGF-beta 3, SPARC, MMP-3, MMP-7, MMP-9, MMP-11, MMP-13, MMP-14 and/or any combinations thereof.

8. Use according to claim 5 **characterised in that** the modification of the physiology of the cells responsible for vascularisation comprises the inhibition of neovascularisation, preferably by inhibiting the proliferation of vascular endothelial cells.

9. Use, according to any of claims 1 to 8, wherein the cancer is selected from any of the group comprising: melanomas, gliomas, carcinomas, adenocarcinomas, leukaemias, lymphomas, myeloma, neoplasms of embryonic or neuroectodermic type and/or sarcomas.

10. Use, according to claim 9, **characterised in that** the carcinoma is selected from any of the following: breast, including ductal, lung, including both small cell and non-small cell, thyroid, including both follicular and papillary, prostate, cervix, endometrium, ovary, bladder, including both endothelial and transitional and/or choriocarcinoma.

11. Use, according to claim 9, **characterised in that** the adenocarcinoma is selected from any of the following: colon, breast, ovary, pancreas, lung, and gastric.

12. Use, according to claim 9, **characterised in that** the leukaemia is selected from: T cell lymphoblastic, B cell lymphoblastic, and T/NK cell leukaemia.

13. Use, according to claim 9, **characterised in that** the lymphoma is selected from: type B, type T, Hodgkin's or Burkitt's lymphoma.

14. Use, according to claim 9, **characterised in that** the sarcomas are preferably osteosarcomas, rhabdomyosarcomas, endometrial stromal sarcomas, fibrosarcomas, malignant fibrous histiocytoma or Edwing sarcoma.

15. Use, according to claim 9, **characterised in that** the neoplasms are preferably neuroblastomas or retinoblastomas.

16. Use, according to any of claims 1 to 15 wherein the antibody recognises at least a sequence of NK1, NK2 or NK3 receptors, selected from: SEQ ID NO:1, SEQ ID NO:2 or SEQ ID NO: 3.

17. Pharmaceutical composition comprising at least one antibody or fragment thereof specific against NK1, NK2 and/or NK3 receptors, or combinations thereof.

18. Composition according to claim 17 wherein the antibody recognises at least a sequence of NK1, NK2 or NK3 receptors, selected from: SEQ ID NO:1, SEQ ID NO:2 or SEQ ID NO: 3.

19. Composition according to any of claims 17 or 18 which further comprises carriers and/or pharmaceutically acceptable vehicles.

20. Use of the pharmaceutical composition of claims 17 to 19 for the treatment of cancer.

21. Use according to claim 20 wherein the cancer is selected from the group comprising: melanoma, gliomas, carcinomas, adenocarcinomas, leukaemias, neoplasms of embryonic or neuroectodermic type and/or sarcomas.

22. Use, according to claim 21, **characterised in that** the carcinoma is selected from any of the following: breast, including ductal, lung, including both small cell and non-small cell, thyroid, including both follicular and papillary, prostate, cervix, endometrium, ovary, bladder, including both endothelial and transitional and/or choriocarcinoma.

23. Use, according to claim 21, **characterised in that** the adenocarcinoma is selected from any of the following: colon, breast, ovary, pancreas, lung, and gastric.

24. Use, according to claim 21, **characterised in that** the leukaemia is selected from: T cell lymphoblastic, B cell lymphoblastic, and T/NK cell leukaemia.

25. Use, according to claim 21, **characterised in that** the lymphoma is selected from: type B, type T, Hodgkin's or Burkitt's lymphoma.

26. Use, according to claim 21, **characterised in that** the sarcomas are preferably osteosarcomas, rhabdomyosarcomas, endometrial stromal sarcomas, fibrosarcomas, malignant fibrous histiocytoma or Edwing sarcoma.

27. Use, according to claim 21, **characterised in that** the neoplasms are preferably neuroblastomas or retinoblastomas.

28. Use of the pharmaceutical composition of claims 17 to 19 for the induction of apoptosis in tumour cells.

29. Use of the pharmaceutical composition of claims 17 to 19 for reducing the tumour size.

30. Use according to claim 29, **characterised in that** the reduction of tumour size is performed by modifying the peritumoural microenvironment.

31. Use according to claim 30 **characterised in that** the modification of the peritumoral microenvironment comprising the modification of the physiology of the stromal matrix and the stromal cells and/or the modification of the physiology of cells involved in inflammatory and immune response and/or the modification of the physiology of the cells responsible for vascularisation.

32. Use according to claim 31 **characterised in that** the modification of the physiology of the stromal matrix and stromal cells and/or the modification of physiology of the cells involved in the inflammatory and immune response comprises the inhibition of tumour markers.

33. Use according to claim 32 wherein the tumour markers are selected preferably from any of the following list: NF-kB, TGF alpha, TGF-beta 1, TGF-beta 2, TGF-beta 3, SPARC, MMP-3, MMP-7, MMP-9, MMP-11, MMP-13, MMP-14 and/or any combinations thereof.

34. Use according to claim 31 **characterised in that** the modification of the physiology of the cells responsible for vascularisation comprises the inhibition of neovascularisation, preferably, by inhibiting the proliferation of vascular endothelial cells.
